(19) Europäisches Patentamt
European Patent Office
Office européen des brevets

(11) Veröffentlichungsnummer: **0 044 979**
**B1**

(12) # EUROPÄISCHE PATENTSCHRIFT

(45) Veröffentlichungstag der Patentschrift: **02.11.83**

(21) Anmeldenummer: **81105233.1**

(22) Anmeldetag: **06.07.81**

(51) Int. Cl.³: **C 07 C 59/68**, C 07 C 69/736, C 07 C 103/34, C 07 C 153/11, A 01 N 39/02

(54) **2-Fluoralkancarbonsäureverbindungen, Verfahren zu ihrer Herstellung, diese enthaltende Herbizide und ihre Anwendung.**

(30) Priorität: **29.07.80 DE 3028627**

(43) Veröffentlichungstag der Anmeldung: **03.02.82 Patentblatt 82/5**

(45) Bekanntmachung des Hinweises auf die Patenterteilung: **02.11.83 Patentblatt 83/44**

(84) Benannte Vertragsstaaten: **AT BE CH DE FR GB IT LI LU NL SE**

(56) Entgegenhaltungen:
**DE - A - 1 593 069**
**DE - A - 2 554 882**
**DE - A - 2 720 654**

(73) Patentinhaber: **BASF Aktiengesellschaft, Carl-Bosch-Strasse 38, D-6700 Ludwigshafen (DE)**

(72) Erfinder: **Varwig, Juergen, Dr., Am Goetzenberg 1, D-6900 Heidelberg (DE)**
Erfinder: **Husslein, Gerd, Dr., Herrenbergstrasse 2, D-6702 Bad Duerkheim (DE)**
Erfinder: **Hamprecht, Gerhard, Dr., Rote-Turm-Strasse 28, D-6940 Weinheim (DE)**
Erfinder: **Wuerzer, Bruno, Dr., Ruedigerstrasse 13, D-6701 Otterstadt (DE)**

**EP 0 044 979 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 2-Fluoralkancarbonsäureverbindungen, Verfahren zu ihrer Herstellung, diese enthaltende Herbizide und ihre Anwendung

Die vorliegende Erfindung betrifft neue fluorierte Aryl- und Heteroaryloxycarbonsäureverbindungen mit herbizider Wirkung, Herbizide, die diese Verbindungen enthalten, Verfahren zur Bekämpfung unerwünschten Pflanzenwuchses mit diesen Verbindungen und Verfahren zu ihrer Herstellung.

Es ist bekannt, Phenoxyalkancarbonsäurederivate – beispielsweise das Dimethylaminsalz der 2-Methyl-4-chlor-phenoxy-a-propionsäure (DE-AS 10 64 286) oder das Dimethylaminsalz der 2,4-Dichlorphenoxy-a-propionsäure (DE-PS15 42 791) als Herbizide zu verwenden.

Die Entdeckung der herbiziden Wirkung von Phenoxyalkancarbonsäuren im Jahre 1942 ermöglichte die Bekämpfung von zahlreichen breitblättrigen Unkräutern in Kulturen von Nutzpflanzen der Familie der Gramineen wie Getreide, Mais oder Reis. Weitere Phenoxyfettsäurederivate mit speziellen Eigenschaften kamen später als Einzelprodukte oder als Mischungspartner hinzu.

In einem umfangreichen Schrifttum werden die Chemie, die Anwendungsbereiche sowie Vor- und Nachteile der bekannten Verbindungen dieser Klasse einschliesslich ihrer Salze und Ester erläutert (Wegler, R. – Chemie der Pflanzenschutz- und Schädlingsbekämpfungsmittel, Bd. 5, 1977). Trotz der weltweiten Anwendung der Phenoxyalkancarbonsäuren ist nicht zu übersehen, dass ihre Anwendung ein gewisses Risiko für die Kulturpflanzen ist. Man versucht dieses durch möglichst präzise Ausbringung der Wirkstoffe zu Zeiten bestimmter wenig empfindlicher Wachstumsstadien der Kulturpflanzen herabzusetzen.

Es wurde nun gefunden, dass 2-Fluoralkancarbonsäure-Verbindungen der allgemeinen Formel

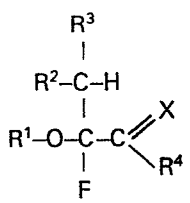

I

worin

$R^1$ einen gegebenenfalls substituierten Aryl- oder Heteroarylrest,

$R^2$ und $R^3$ unabhängig voneinander Wasserstoff, einen gegebenenfalls substituierten Alkyl-, Cycloalkyl-, Aralkyl-, Aryl- oder Heteroarylrest,

X Sauerstoff oder Schwefel und

$R^4$ eine Hydroxylgruppe oder die Gruppen $OR^5$, $SR^5$ oder 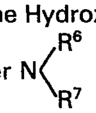 bedeuten, worin

$R^5$ einen gegebenenfalls substituierten Alkyl-, Alkenyl-, Alkinyl-, Cycloalkyl-, Cycloalkenyl-, Alkanon-, Aryl-, Heteroaryl- oder Heteroarylalkylrest,

$R^6$ Wasserstoff oder die für $R^5$ genannten Reste, ausser einem gegebenenfalls substituierten m-Anilinorest,

$R^7$ unabhängig von $R^6$ die für $R^5$ genannten Reste oder eine Hydroxylgruppe oder einen gegebenenfalls substituierten Alkoxy- oder Alkylthiorest, eine Cyano- oder Cyanoalkylgruppe oder den Rest 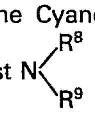 bedeutet, worin $R^8$ und $R^9$ unabhängig voneinander Wasserstoff oder einen gegebenenfalls substituierten Alkyl- oder Arylrest bedeuten und ferner $R^6$ und $R^7$ zusammen mit dem benachbarten Stickstoffatom einen gegebenenfalls substituierten heterocyclischen Rest bedeuten, der gegebenenfalls eine oder mehrere Doppelbindungen enthält oder zusätzlich zu dem Stickstoffatom ein oder mehrere Heteroatome enthält, oder die Salze dieser Verbindungen eine verbesserte herbizide Wirkung gegen einzelne breitblättrige Unkrautarten und gleichzeitig eine günstigere Verträglichkeit für Kulturpflanzen besitzen als bekannte substituierte Phenoxyalkancarbonsäurederivate.

Die Verbindungen der allgemeinen Formel I werden beispielsweise hergestellt, indem man

a) eine Acrylsäureverbindung der allgemeinen Formel

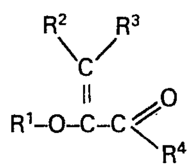

II

worin $R^1$, $R^2$, $R^3$ und $R^4$ die oben genannten Bedeutungen haben, mit Flusssäure mit oder ohne Verdünnungsmittel bei Temperaturen zwischen −80 °C und +150 °C, gegebenenfalls in Gegenwart eines Katalysators umsetzt und die so erhaltenen Verbindungen gegebenenfalls schwefelt oder

b) eine 2-Fluoralkancarbonsäure der Formel

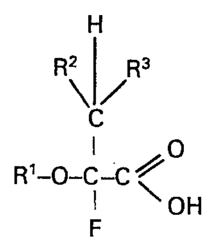

III

oder deren Salze mit Thionylchlorid, Phosgen, Phosphorpentachlorid, N,N'-Carbonyldiimidazol, N,N'-Carbonylditriazol, N,N'-Thionyldiimidazol oder N,N'-Thionylditriazol oder deren Mischung gegebenenfalls in Gegenwart eines Verdünnungsmittels umsetzt und danach mit einer Verbindung der Formel

$R^4H$ IV

worin $R^4$ die oben genannten Bedeutungen ausser einer Hydroxylgruppe hat, gegebenenfalls in Gegenwart eines Verdünnungsmittels, einer anorganischen oder organischen Base oder eines Reaktionsbeschleunigers umsetzt und die so erhaltene Verbindung gegebenenfalls schwefelt.

Die neuen Stoffe treten in enantiomeren Formen auf. Auch deren Mischung sowie die optisch angereicherten oder reinen Formen werden von den Patentansprüchen umfasst. Die reinen optischen Isomeren können ggf. aus dem Racemat durch die üblichen Trennmethoden, beispielsweise durch fraktionierte Kristallisation diastereomerer Salze mit optisch aktiven Basen, hergestellt werden.

$R^1$ bedeutet beispielsweise einen gegebenenfalls substituierten Arylrest mit 6 bis 20 Kohlenstoffatomen, insbesondere gegebenenfalls substituiertes Phenyl, Naphthyl; einen gegebenenfalls substituierten Heteroarylrest mit 5 bis 7, insbesondere 6 Ringatomen, von denen 1 bis 3, insbesondere 1 bis 2 Heteroatome sind, die gleich oder verschieden sein können und vorzugsweise Sauerstoff, Schwefel oder Stickstoff sind, beispielsweise einen gegebenenfalls substituierten Pyridyl- oder Pyrimidinylrest.

$R^2$ und $R^3$ bedeutet beispielsweise Wasserstoff, geradkettiges, gegebenenfalls substituiertes Alkyl mit 1 bis 10, insbesondere 1 bis 6 Kohlenstoffatomen, verzweigtes, gegebenenfalls substituiertes Alkyl mit 3 bis 8, insbesondere 3 bis 6 Kohlenstoffatomen, beispielsweise Methyl, Ethyl, n-Propyl, iso-Propyl, n-Butyl, iso-Butyl, tert.-Butyl, n-Pentyl, 2-Pentyl, 3-Pentyl, 4-Methyl-3-pentyl, 1-Chlorethyl, Trifluormethyl, Trichlormethyl, Difluormethyl, Nitromethyl, Cyanomethyl, 2-Chlorethyl, 1-Chlorpropyl, 2-Chlorpropyl, 1-Chlor-2-propyl, 1-Fluorethyl, 2-Fluorethyl, 1-Fluor-2-propyl, 3-Brombutyl, 1,1,1-Trifluorisopropyl, Methoxyethyl, Methoxyisopropyl, Ethoxy-tert.-butyl, Methoxy-tert.-butyl, gegebenenfalls substituiertes Cycloalkyl mit 3 bis 7, insbesondere 3 bis 6 Kohlenstoffatomen im Ring, beispielsweise Cyclopropyl, Cyclobutyl, Cyclopentyl, Cyclohexyl, 2-Chlorcyclohexyl, 2-(Trifluormethyl)-cyclohexyl; einen gegebenenfalls substituierten Aralkylrest mit 6 bis 10 Kohlenstoffatomen im Arylteil und 1 bis 2 Kohlenstoffatomen im Alkylteil, insbesondere gegebenenfalls substituiertes Benzyl, α-Phenylethyl; einen gegebenenfalls substituierten Arylrest mit 6 bis 20 Kohlenstoffatomen, insbesondere gegebenenfalls substituiertes Phenyl, Naphthyl; einen gegebenenfalls substituierten Heteroarylrest mit 5 bis 7, insbesondere 5 bis 6 Ringatomen, von denen 1 bis 3, insbesondere 1 bis 2 Heteroatome sind, die gleich oder verschieden sein können und vorzugsweise Sauerstoff, Schwefel oder Stickstoff sind, beispielsweise einen gegebenenfalls substituierten Pyridyl-, Pyrazolyl-, Thienyl-, Furyl- oder Pyrimidinylrest.

$R^5$ bedeutet beispielsweise einen unverzweigten Alkylrest mit 1 bis 20 Kohlenstoffatomen, einen verzweigten Alkylrest mit 3 bis 15 Kohlenstoffatomen, einen geradkettigen oder verzweigten Alkenyl- oder Alkinylrest mit 3 bis 15 Kohlenstoffatomen oder einen cyclischen Alkyl- oder Alkenylrest mit 3 bis 10 Ringkohlenstoffatomen oder einem Alkanonrest, wobei die Reste jeweils gegebenenfalls substituiert sein können. Als Beispiele für $R_5$ seien genannt: Methyl, Ethyl, n-Propyl, Isopropyl, Cyclopropyl, n-Butyl, Isobutyl, tert-Butyl, Cyclobutyl, n-Pentyl, 2-Pentyl, 3-Pentyl, tert-Amyl, Neopentyl, 2-Methyl-2-butyl, 3-Methyl-butyl, 3-Methyl-2-butyl, Cyclopentyl, n-Hexyl, 4-Methyl-2-pentyl, 2,3-Dimethylbutyl, 2-Methyl-1-pentyl, 2-Hexyl, 3-Methyl-2-pentyl, 3-Methylpentyl, 4-Methylpentyl, 3-Methyl-3-pentyl, 4,4-Dimethylbutyl, Cyclohexyl, Heptyl, 2-Heptyl, 3-Heptyl, 4-Heptyl, Cycloheptyl, 1-Octyl, 2-Octyl, 3-Octyl 4-Octyl, 5-Octyl, 5-Ethyl-2-heptyl, 2,6-Dimethyl-4-heptyl, 7-Ethyl-2-methyl-4-nonyl, 2,4-Dimethyl-3-pentyl, 3-Methyl-2-heptyl, 5-Ethyl-2-nonyl, Nonyl, Decyl, Undecyl, Dodecyl, Tridecyl, Tetradecyl, Pentadecyl, Hexadecyl, Heptadecyl, Octadecyl, Nonadecyl, Eicosyl, 6-Ethyl-3-decyl, 6-Ethyl-3-octyl, 2-Methyl-2-pentyl, 2,3-Dimethyl-2-butyl, 2-Methyl-2-hexyl, 3-Ethyl-3-pentyl, 3-Methyl-3-hexyl, 2,3-Dimethylpentyl-3, 2,4-Dimethyl-2-pentyl, 2,2,3-Trimethyl-3-butyl, 2-Methyl-2-heptyl, 4-Methyl-4-heptyl, 2,4-Dimethyl-2-octyl, 1-Methyl-1-cyclopentyl, 1-Methyl-1-cyclohexyl, 1-Ethyl-1-cyclohexyl, Chlor-tert.-butyl, 1,1-Dichlor-2-methyl-2-propyl, 1,3-Dichlor-2-methyl-2-propyl, 1-Cyclohexyl-1-ethyl, 1-Chlorethyl, 2-Chlorpropyl, 3-Chlorpropyl, 1-Chlor-2-propyl, 2-Chlorbutyl, 2-Chlor-2-methyl-3-propyl, 2-Fluorethyl, 2-Fluorpropyl, 3-Fluorpropyl, 1-Fluor-2-propyl, 2-Fluorbutyl, 2-Fluor-2-methyl-3-propyl, 2-Bromethyl, 3-Brompropyl, 4-Chlorbutyl, 2-Chlorcyclohexyl, 1,1,1-Trifluorisopropyl, Hexafluor-2-methyl-isopropyl, Hexafluorisopropyl, Hexachlorisopropyl, 1,2-Dibromallyl, 2,2,2-Trifluorethyl, 1-Chlor-butin-2-yl-4, 3-Chlor-butin-1-yl-4, 1-Chlor-buten-2-yl-4, 2,2,2-Trichlorethyl, 1-Chlorpentin-2-yl-4, 2,2,2-Tribromethyl, 3,4,4-Trichlorbuten-3-yl-2, 1-Brom-2-propyl, 1,3-Dibrom-2-propyl, 3-Chlorbuten-1-yl-4, Allyl, Propargyl, Crotyl, Butin-2-yl-1, Methallyl, Buten-1-yl-3, Butin-1-yl-3, Buten-1-yl-4, Butin-1-yl-4, 2-Methylbuten-1-yl-3, 2-Methylbuten-2-yl-1, 2-Methylbuten-2-yl-4, 3-Methyl-buten-1-yl-3, 3-Methyl-buten-1-yl-3, 2-Methylbuten-1-yl-4, 2-Ethylhexen-2-yl-1, Hexen-5-yl-1, Undecen-10-yl-1, 1-Ethinyl-cyclohexyl, Methoxyethyl, Ethoxyethyl, 3-Methoxypropyl, Methoxyisopropyl, 3-Methoxybutyl, 1-Methoxy-butyl-2, Ethoxy-tert-butyl, Methoxy-tert-butyl, Cyclohexyloxy-tert-butyl, 2-Methoxy-butyl, 4-Methoxy-butyl, β-Methyl-mercapto-ethyl, β-Ethylmercaptoethyl, 3-Methyl-mercapto-propyl, 1-Methylmercaptobutyl-2, Methylmercaptotert-butyl, 2-Methylmercaptobutyl, 4-Methyl-mercaptobutyl, 2-n-Butoxyethyl, 2-Ethoxypropyl, 3-Ethoxy-2-propyl, 2-Methyl-butanon-3-yl-2, 2-Methyl-pentanon-4-yl-2, 3-Butanon-1-yl, 3-Butanon-2-yl, 2-Propanon-1-yl, 2-Pentanon-1-yl, N,N-Dimethylaminoethyl; $R^5$ bedeutet beispielsweise einen gegebenenfalls substituierten Aryl-, Arylalkyl-, Heteroaryl- oder Heteroarylalkylrest mit 6 bis 12 Kohlenstoffatomen im Aryl- bzw. Heteroarylteil und 1 bis 3 Kohlenstoffatomen im Alkylteil, beispielsweise einen gegebenenfalls substituierten Phenyl- oder Naphthylrest oder einen gegebenenfalls substituierten Heteroarylrest mit 5 bis 6 Ringatomen und 1 bis 3, insbesondere 1 bis 2 Heteroatomen im Ring, die gleich oder verschieden sein können und vorzugsweise Sauerstoff, Stickstoff oder Schwefel sind, beispielsweise einen Pyridyl-, Pyrazolyl- oder Pyrimidinylrest.

Als bevorzugte Substituenten des Aryl- oder Heteroarylrestes seien genannt: Halogen, insbesondere Fluor, Chlor oder Brom; Nitro, ein geradkettiger Alkylrest mit 1 bis 14, ein verzweigter oder cyclischer Alkylrest mit 3 bis 14 Kohlenstoffatomen; ein Alkoxy- oder Alkylthiorest mit 1 bis 5 Kohlenstoffatomen, ein Halogenalkyl-, Halogenalkoxy-, Halogenalkylthio- oder Halogenalkylsulfonylrest mit bis zu 4 Kohlenstoffatomen und bis zu 9 Halogenatomen, insbesondere Fluor oder Chlor; eine Formyl-, Cyano- oder Thiocyanatogruppe; ein Alkoxycarbonyl-, Dialkylketo-, Alkylcarbonyl-, Alkyl- und Dialkylcarbomoyl-, Acylamino- oder ein Alkylaminosulfonylrest mit 2 bis 10 Kohlenstoffatomen, beispielsweise ein Methoxycarbonyl-, Acetonyl-, Propionyl-, N,N-Dimethylcarbomoyl- oder ein Butyrylaminorest; ein Alkoxyalkyl- oder Alkylthioalkylrest mit 2 bis 6 Kohlenstoffatomen, beispielsweise ein Ethoxypropyl- oder ein Methylmercaptomethylrest; ein gegebenenfalls substituierter Phenyl- oder Phenylcarbonylrest mit 6 bis 11 Kohlenstoffatomen; ein gegebenenfalls insbesondere in der 4-Stellung substituierter Aryloxy-, Arylalkyloxy- oder Arylazorest, beispielsweise ein 4-(4'-Chlorphenoxy)-, 4-(2', 4'-Dichlorphenoxy)-, 4-(4'-Trifluormethylphenoxy)-Rest oder ein 4-(4'-Chlorphenylazo)- oder 4-(3'-Fluorphenylazo)-Rest; ein gegebenenfalls substituierter Arylthio-, Arylsulfinyl- oder Arylsulfonylrest; ein gegebenenfalls substituierter Arylalkylrest, insbesondere ein unsubstituierter oder substituierter Benzylrest; ein geradkettiger Alkenylrest mit 2 bis 6 oder ein verzweigter Alkenylrest mit 3 bis 7 Kohlenstoffatomen; ein geradkettiger Alkinylrest mit 2 bis 4 oder ein verzweigter Alkinylrest mit 3 bis 6 Kohlenstoffatomen; ankondensierte Ringe, insbesondere aus den Klassen der Aromaten und Heterocyclen, beispielsweise ankondensierte gegebenenfalls substituierte Benzo-, Benzofuran- oder Peridinoreste.

$R^6$ und $R^7$ können zusammen mit dem benachbarten Stickstoffatom einen gegebenenfalls substituierten heterocylischen Rest bedeuten, beispielsweise einen 3- bis 7-gliedrigen Heterocyclus, der gegebenenfalls eine oder mehrere Doppelbindungen sowie weitere Heteroatome wie Stickstoff, Sauerstoff oder Schwefel enthält, beispielsweise einen Pyrrolidinyl-, 4-Methylpiperazinyl-, Imidazolyl-, 1,2,4-Triazolyl-, Piperidinyl- oder 2,6-Dimethylmorpholinylrest.

Salze sind beispielsweise die Alkali- (Na, K) oder Erdalkalisalze (Mg, Ca) der Carbonsäuren.

Die Umsetzung wird zweckmässig bei einer Temperatur von –80 bis +120 °C, vorzugsweise von –75 bis +80 °C, insbesondere von –70 bis +30 °C, drucklos oder unter Druck, kontinuierlich oder diskontinuierlich durchgeführt. Zweckmässig verwendet man unter den Reaktionsbedingungen inerte Lösungsmittel. Als Lösungsmittel kommen z.B, in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Chlorkohlenwasserstoffe, z.B. Tetrachloräthylen, 1,1,2,2- oder 1,1,1,2-Tetrachloräthan, Amylchlorid, Dichlorpropan, Methylenchlorid, Dichlorbutan, Isopropylbromid, Chloroform, Äthyljodid, Propyljodid, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, o-, p- und m-Dichlorbenzol, o-, m-, p-Chlortoluol, 1,2,4-Trichlorbenzol; Ketone wie Methyläthylketon, Aceton, Diisopropylketon, Diäthylketon, Methylisobutylketon, Mesityloxid, Acetophenon, Cyclohexanon, Äthylisoanylketon, Diisobutylketon, Methylcyclohexanon, Dimethylcyclohexanon; Ester wie Methylacetat, n-Propylacetat, Methylpropionat, Butylacetat, Äthylformiat, Phthalsäuremethylester, Benzoesäuremethylester, Essigester, Phenylacetat; Nitrile wie Acetonitril, Butyronitril, Isobutyronitril, Benzonitril, m-Chlorbenzonitril; Sulfoxide wie Dimethylsulfoxid, Diäthylsulfoxid, Dimethylsulfon, Diäthylsulfon, Methyläthylsulfon, Tetramethylensulfon; aliphatische oder cycloaliphatische Kohlenwasserstoffe, z.B. Heptan, α-Pinen, Pinan, Nonan, Benzinfraktionen innerhalb eines Siedepunktintervalls von 70 bis 190 °C, Cyclohexan, Methylcyclohexan, Dekalin, Petroläther, Hexan, Ligroin, 2,2,4-Trimethylpentan, 2,2,3-Trimethylpentan, 2,3,3-Trimethylpentan, Octan; N,N-disubstituierte Säureamide wie Dimethylformamid, Dimethylacetamid; und entsprechende Gemische. Zweckmässig verwendet man das Lösungsmittel in einer Menge von 100 bis 10 000 Gewichtsprozent, vorzugsweise von 500 bis 2 000 Gewichtsprozent, bezogen auf Ausgangsstoff.

Die Umsetzung kann in Gegenwart eines Reaktionsbeschleunigers als Katalysator, vorteilhaft in einer Menge von 0,001 bis 2, vorzugsweise von 0,001 bis 0,1 Äquivalenten Beschleuniger, bezogen auf ein Mol Ausgangsstoff II, durchgeführt werden.

Als Reaktionsbeschleuniger kommen in Frage: Aktivkohle, Bortrifluorid, Aluminium- oder Antimonhalogenide wie Chloride oder Bromide, Eisenchlorid, Zinkchlorid, Aluminiumoxid, Titan-, Tantal-, Niob-, Rhenium- oder Molybdänhalogenide wie Chloride oder Bromide oder Oxychloride.

Man verwendet im allgemeinen auf 1 Mol des Ausgangsstoffes II 0,8 bis 20 Mol, vorzugsweise 1 bis 15 Mol Flusssäure. Soll jedoch die Flusssäure und/oder der Katalysator als Lösungsmittel angewendet werden, können die angegebenen Molverhältnisse erheblich überschritten werden, zweckmässig sind dann Mengen von 10 bis 100, insbesondere 10 bis 30 Mol Fluorwasserstoff je Mol Ausgangsstoff II und/oder zweckmässig 0,001 bis 2, insbesondere 0,001 bis 0,1 Äquivalente Beschleuniger je Mol Ausgangsstoff II.

Die Reaktion kann wie folgt durchgeführt werden: Ein Gemisch von Ausgangsstoff und Fluorwasserstoff, gegebenenfalls zusammen mit Lösungsmittel, wird während 0,1 bis 60 Stunden bei

der Reaktionstemperatur gehalten. Aus dem Reaktionsgemisch wird der Endstoff I in üblicher Weise, z.B. durch Filtration und fraktionierte Destillation abgetrennt. Man kann aber das Reaktionsgemisch auch auf Eis giessen und die organische Phase abtrennen bzw. mit einem mit Wasser nicht mischbaren organischen Lösungsmittel extrahieren.

In einer bevorzugten Form des erfindungsgemässen Verfahrens legt man bei –80 bis –40 °C wasserfreie Flusssäure vor und gibt langsam den Ausgangsstoff II, insbesondere eine Lösung desselben in einem indifferenten Lösungsmittel, zu und führt die Reaktion während 10 Minuten bis 15 Stunden, insbesondere während 10 Minuten bis 5 Stunden, durch. Die Reihenfolge der Zugabe der Reaktanten kann auch umgekehrt werden, indem man den gasförmigen Fluorwasserstoff dem Reaktionspartner zugibt.

Die Verbindungen der Formel I besitzen eine selektive herbizide Wirksamkeit. Sie sind daher für den Gebrauch als Pflanzenschutzmittel zur Bekämpfung von Schadpflanzen in landwirtschaftlichen Kulturen geeignet.

Beispiel 1

In einem Rührkolben (fluoriertes Polyethylen) werden zu einer Lösung von 17 Teilen (Gew.-Teilen) 2-(4'-Fluorphenoxy)-acrylsäuremethylester in 100 Teilen Tetrachlorkohlenstoff unter Rühren und Kühlung auf –65 °C 10 Teile wasserfreie Flusssäure zugesetzt. Die Mischung wird auf Raumtemperatur erwärmt und das Lösungsmittel und nicht verbrauchte Flusssäure im Vakuum über einen Kühler abdestilliert.

Die Destillation des Rückstandes ergibt 13 Teile (70% d. Th.) 2-Fluor-2-(4'-fluorphenoxy)-propionsäuremethylester. $Kp_{0,05}$ 56 °C.

Beispiel 2

In einem Rührkolben werden zu einer Lösung von 29 Teilen 2-(2',4'-Dichlorphenoxy)-acrylsäuremethylester in 100 Teilen Petrolether unter Rühren und Kühlung auf –78 °C 30 Teile wasserfreie Flusssäure zugesetzt. Die Reaktionsmischung lässt man sich auf Raumtemperatur erwärmen und destilliert unter schwachem Vakuum das Lösungsmittel und den überschüssigen Fluorwasserstoff ab. Der Rückstand wird in Petrolether gelöst, Natriumfluorid zugesetzt und abfiltriert.

Die Destillation des Filtrats ergibt 24 Teile (77% d.Th.) 2-(2',4'-Dichlorphenoxy)-2-fluor-propionsäuremethylester. $Kp_{0,1}$ 90 °C.

Beispiel 3

In einem Rührkolben werden 89 Teile 2-(2',4'-Dichlorphenoxy)-acrylsäure in 100 Teilen Methylenchlorid gelöst und 80 Teile Fluorwasserstoff unter Rühren und Kühlung auf –65 °C zugesetzt. Die Mischung wird auf Raumtemperatur erwärmt und überschüssiger Fluorwasserstoff abdestilliert. Das Rohprodukt wird mit n-Hexan gerührt und abfiltriert. Ausbeute: 86 Teile (89% d.Th.) 2-(2',4'-Dichlorphenoxy)-2-fluorpropionsäure. Fp.: 88–90 °C.

Beispiel 4

In einem Rührkolben werden zu 14 Teilen 2-(2',4'-Dichlorphenoxy)-acrylsäure bei –65 °C 30 Teile wasserfreie Flusssäure zugesetzt, unter Rühren auf Raumtemperatur erwärmt und anschliessend der überschüssige Fluorwasserstoff abdestilliert. Das Produkt wird aus n-Hexan umkristallisiert. Ausbeute: 9 Teile (59% d.Th.) 2-(2',4'-Dichlorphenoxy)-2-fluorpropionsäure. Fp.: 88–90 °C.

Das Herstellungsverfahren a wird durch die Beispiele der Tabelle I weiter verdeutlicht.

Tabelle I

| Beispiel | Teile Ausgangsstoff II | Teile HF | Lösungsmittel | Teile Lösungsmittel | $R^1$ | $R^2$ | $R^3$ | $R^4$ . . . Forts. nächste Seite |
|---|---|---|---|---|---|---|---|---|
| 5 | 28 | 30 | $CH_2Cl_2$ | 100 | 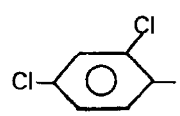 | H | H | $-O-i-C_8H_{17}$ |
| 6 | 45 | 50 | $CH_2Cl_2$ | 100 | 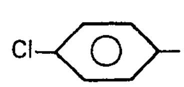 | H | H | $-OCH_3$ |
| 7 | 25 | 20 | Petrolether | 100 | 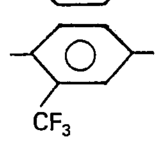 | H | H | $-OCH_3$ |
| 8 | 35 | 30 | $CH_2Cl_2$ | 100 | 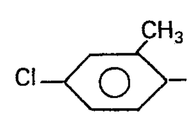 | H | H | $-OC_2H_5$ |
| 9 | 22 | 25 | $CH_2Cl_2$ | 100 | 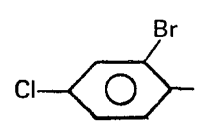 | H | H | $-OCH_3$ |

Tabelle I: Fortsetzung

| Beispiel | Teile Ausgangsstoff II | Teile HF | Lösungsmittel | Teile Lösungsmittel | $R^1$ | $R^2$ | $R^3$ | $R^4$ |
|---|---|---|---|---|---|---|---|---|
| 10 | 28 | 20 | $CH_2Cl_2$ | 100 | 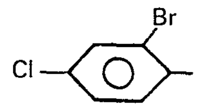 | H | H | –OH |
| 11 | 32 | 35 | $CH_2Cl_2$ | 100 | 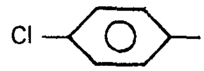 | H | H | –OH |

| Beispiel | Kp (mbar) °C | $n_D^{22}$ | Endstoff Fp °C | Ausbeute % der Theorie |
|---|---|---|---|---|
| zu 5 | | 1.4860 | | 70 |
| zu 6 | 40 °C (0,1) | | | 62 |
| zu 7 | 45 °C | | | 75 |
| zu 8 | 100 °C (0,2) | | | 60 |
| zu 9 | 101 °C (0,3) | | | 71 |
| zu 10 | | | 90–91 °C | 79 |
| zu 11 | | | 52 °C | 45 |

Beispiel 12

Zu einer Lösung von 20,4 Teilen Imidazol in 150 Teilen Tetrahydrofuran tropft man bei 10 bis 20 °C 8,9 Teile Thionylchlorid. Man rührt 30 min. bei Raumtemperatur nach, saugt ab und versetzt das Filtrat mit 19 Teilen 2-(2',4'-Dichlorphenoxy)-2-fluorpropionsäure. Man rührt 2 Stunden bei Raumtemperatur nach und gibt dann 5,1 Teile Propanthiol-2 zu und rührt 6 Stunden bei Raumtemperatur nach. Nach dem Einengen nimmt man die Reaktionsmischung in Methylenchlorid auf, wäscht zweimal mit Wasser, trocknet die organische Phase mit Magnesiumsulfat und zieht das Lösungsmittel ab. Man erhält so 21 Teile (90% d.Th.) 2-(2',4'-Dichlorphenoxy)-2-fluorpropionsäure-isopropylthio-ester; $n_D^{22}$ = 1,5272.

Als weitere herbizide Wirkstoffe der allgemeinen Formel seien beispielhaft aufgeführt:

| Beispiel Nummer | $R^1$ | $R^2$ | $R^3$ | X | $R^4$ | Fp, Kp [%C] |
|---|---|---|---|---|---|---|
| 13 | 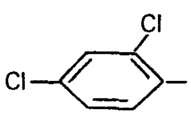 | H | H | O | 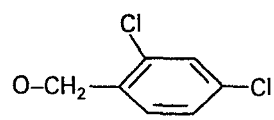 | |
| 14 | do. | H | H | O | 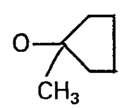 | $n_D^{20}$ =1,5070 |
| 15 | do. | H | H | O | 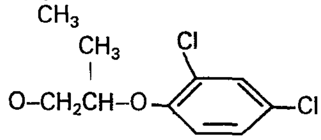 | |
| 16 | do. | H | H | O | 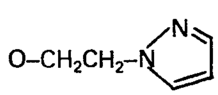 | |
| 17 | do. | H | H | O | $O–C_2H_5$ | |
| 18 | do. | H | H | O | 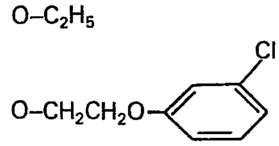 | $n_D^{22}$ =1,5492 |
| 19 | do. | H | H | O | $N(C_2H_5)_2$ | |
| 20 | do. | H | H | O | 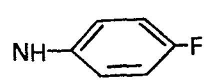 | |

(Fortsetzung)

| Bei-spiel Nummer | $R^1$ | $R^2$ | $R^3$ | X | $R^4$ | Fp, Kp [%C] |
|---|---|---|---|---|---|---|
| 21 | 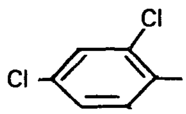 | H | H | O | NH– 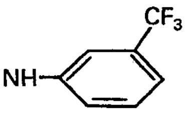 | |
| 22 | do. | H | H | O | NH 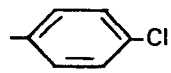 | |
| 23 | do. | H | H | O | NH–tert.$C_4H_9$ | 88–90 |
| 24 | do. | H | H | O | $-SCH_3$ | |
| 25 | do. | H | H | O | S– 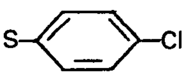 | 45– 48 |
| 26 | do. | H | H | O | $S-CH_2-$ 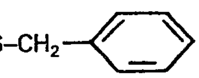 | $n_D^{20}$ =1,5826 |
| 27 | do. | H | H | O | S– 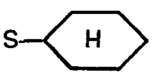 | $n_D^{20}$ =1,5465 |
| 28 | do. | H | $CH_3$ | O | $O-CH_3$ | |
| 29 | do. | $CH_3$ | $CH_3$ | O | $O-C_2H_5$ | |
| 30 | do. | $CH_2-$ 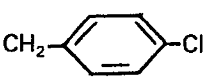 | H | O | $O-CH_3$ | |
| 31 | 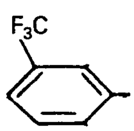 | H | H | O | $O-i-C_8H_{17}$ | |
| 32 | do. | H | H | O | $OC_2H_5$ | |
| 33 | do. | H | H | O | O– 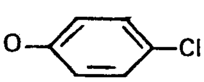 | |
| 34 | 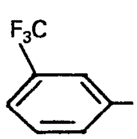 | H | H | O | $O-CH_2-$ 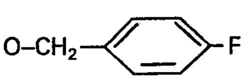 | |
| 35 | do. | H | H | O | $S-CH_3$ | |
| 36 | do. | H | H | O | S 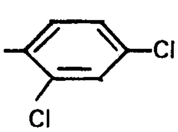 | |

| Beispiel Nummer | $R^1$ | $R^2$ | $R^3$ | X | $R^4$ | Fp, Kp [%C] |
|---|---|---|---|---|---|---|
| 37 | 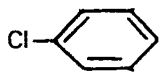 | $CH_3$ | H | O | $OCH_3$ | |
| 38 | do. | $CH_3$ | $CH_3$ | O | $OCH_3$ | |
| 39 | do. | H | H | O | $OC_2H_5$ | |
| 40 | do. | H | H | O | $O–i–C_8H_{17}$ | $n_D^{22} = 1,4812$ |
| 41 | do. | H | H | O | 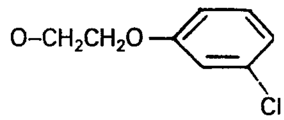 | |
| 42 | do. | H | H | O | $N(CH_3)_2$ | |
| 43 | do. | H | H | O | 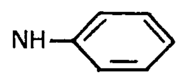 | |
| 44 | do. | H | H | O | 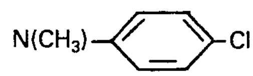 | |
| 45 | do. | H | H | O | $N(CH_2CH{=}CH_2)_2$ | |
| 46 | 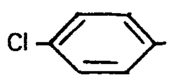 | H | H | O | $S–C_3H_7$ | |
| 47 | 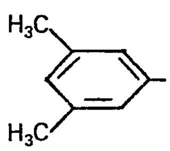 | H | H | O | $OCH_3$ | |
| 48 | do. | H | H | O | $NH–tert.C_4H_9$ | |
| 49 | do. | H | H | O | $NH–C(CH_3)_2(C{\equiv}CH$ | |
| 50 | 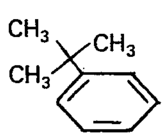 | H | H | O | $OCH_3$ | |
| 51 | do. | H | H | O | $OC_2H_5$ | |
| 52 | do. | H | H | O | $NH–tert.C_4H_9$ | |
| 53 | 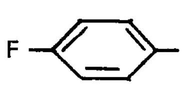 | H | H | O | $OC_2H_5$ | |
| 54 | do. | H | H | O | $O–i–C_3H_7$ | |

(Fortsetzung)

| Beispiel Nummer | $R^1$ | $R^2$ | $R^3$ | X | $R^4$ | Fp, Kp [%C] |
|---|---|---|---|---|---|---|
| 55 | 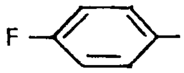 | H | H | O | O–$CH_2CH_2O$– 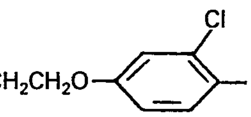 | |
| 56 | do. | H | H | O | OH | |
| 57 | do. | H | H | O | O–$CH_2CH_2O$– 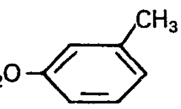 | |
| 58 | do. | H | H | O | O–tert.$C_4H_9$ | |
| 59 | do. | H | H | O | $SCH_3$ | |
| 60 | do. | H | H | O | $SC_2H_5$ | |
| 61 | do. | H | H | O | 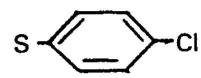 | |
| 62 | do. | H | H | O | $N(CH_3)_2$ | |
| 63 | do. | H | H | O | 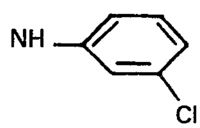 | |
| 64 | 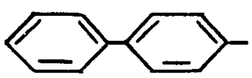 | H | H | O | $OCH_3$ | |
| 65 | do. | H | H | O | $OC_2H_5$ | |
| 66 | do. | H | H | O | O–$CH_2$– 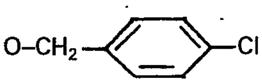 | |
| 67 | do. | H | H | O | 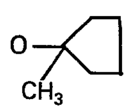 | |
| 68 | do. | H | H | O | $SCH_3$ | |
| 69 | do. | H | H | O | $SC_2H_5$ | |
| 70 | do. | H | H | O | S–tert.$C_4H_9$ | |
| 71 | do. | H | H | O | OH | |
| 72 | do. | $CH_3$ | H | O | $OCH_3$ | |
| 73 | do. | H | H | O | $N(CH_3)_2)$ | |

(Fortsetzung)

| Beispiel Nummer | $R^1$ | $R^2$ | $R^3$ | X | $R^4$ | Fp, Kp [°C] |
|---|---|---|---|---|---|---|
| 74 | 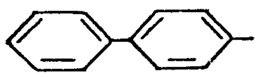 | H | H | O | 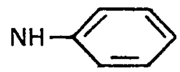 | |
| 75 | do. | H | H | O | NH–tert.$C_4H_9$ | |
| 76 | 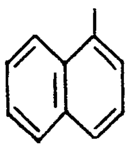 | H | H | O | OH | |
| 77 | do. | H | H | O | $OC_2H_5$ | |
| 78 | do. | H | H | O | S–i–$C_3H_7$ | |
| 79 | do. | H | H | O | 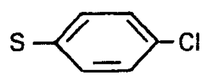 | |
| 80 | do. | H | H | O | $N(CH_3)_2$ | |
| 81 | 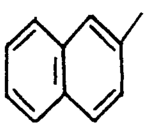 | H | H | O | $NHCH_3$ | |
| 82 | do. | H | H | O | 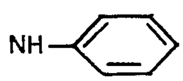 | |
| 83 | do. | H | H | O | $OCH_3$ | |
| 84 | do. | H | H | O | $OC_2H_5$ | |
| 85 | do. | H | H | O | $SCH_3$ | |
| 86 | do. | H | H | O | 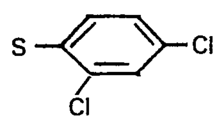 | |
| 87 | do. | H | H | O | 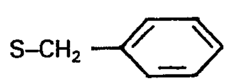 | |
| 88 | 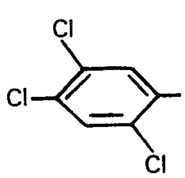 | H | H | O | OH | |
| 89 | do. | H | H | O | $OC_2H_5$ | |
| 90 | do. | H | H | O | O–i–$C_3H_7$ | |
| 91 | do. | H | H | O | 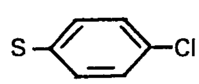 | |

(Fortsetzung)

| Beispiel Nummer | $R^1$ | $R^2$ | $R^3$ | X | $R^4$ | Fp, Kp [%C] |
|---|---|---|---|---|---|---|
| 92 | 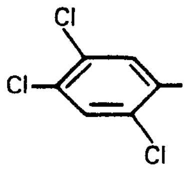 | H | H | O | S 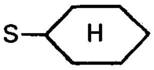 | |
| 93 | do. | H | H | O | $SCH_3$ | |
| 94 | do. | H | H | O | $N(CH_3)_2$ | |
| 95 | do. | H | H | O | NH 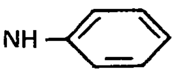 | |
| 96 | do. | H | H | O | $N(CH_3)$ 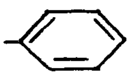 | |
| 97 | do. | H | H | O | $NHCH_2$ 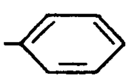 | |
| 98 | 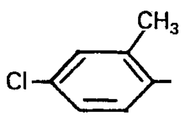 | H | H | O | OH | 76–77 |
| 99 | do. | H | H | O | $OCH_3$ | |
| 100 | do. | H | H | O | $O–i–C_3H_7$ | |
| 101 | do. | H | H | O | $O–CH_2CH_2–O$ 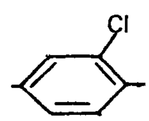 | |
| 102 | do. | H | H | O | $O–CH_2CH_2O$ 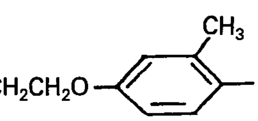 | |
| 103 | do. | H | H | O | $SCH_3$ | |
| 104 | do. | H | H | O | $SC_2H_5$ | |
| 105 | do. | H | H | O | S 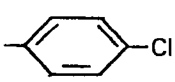 | |
| 106 | do. | H | H | O | $NHCH_3$ | |
| 107 | do. | H | H | O | NH 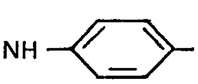 | |
| 108 | do. | H | H | O | $N(C_2H_5)_2$ | |

(Fortsetzung)

| Beispiel Nummer | $R^1$ | $R^2$ | $R^3$ | X | $R^4$ | Fp, Kp [°C] |
|---|---|---|---|---|---|---|
| 109 | 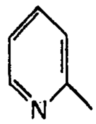 | H | H | O | $OCH_3$ | |
| 110 | 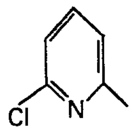 | H | H | O | $OCH_3$ | |
| 111 | 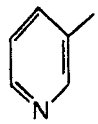 | H | H | O | $OCH_3$ | |
| 112 | 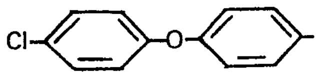 | H | H | O | OH | |
| 113 | do. | H | H | O | $OCH_3$ | |
| 114 | do. | H | H | O | 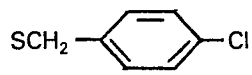 | |
| 115 | do. | H | H | O | $N(CH_3)_2$ | |
| 116 | 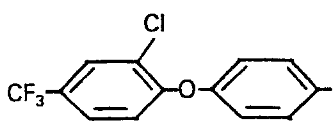 | H | H | O | $OCH_3$ | |
| 117 | do. | H | H | S | $NH_2$ | |
| 118 | do. | H | H | O | $SC_2H_5$ | |
| 119 | do. | H | H | O | OH | |
| 120 | 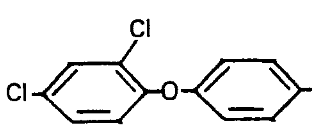 | H | H | O | $OCH_3$ | |
| 121 | 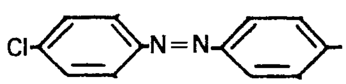 | H | H | O | $OCH_3$ | |
| 122 | 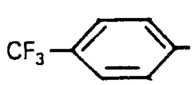 | H | H | O | 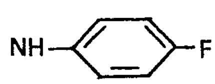 | |
| 123 | do. | H | H | O | OH | |
| 124 | 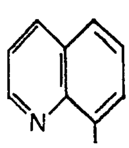 | H | H | O | $OCH_3$ | |

(Fortsetzung)

| Beispiel Nummer | $R^1$ | $R^2$ | $R^3$ | X | $R^4$ | Fp, Kp [°C] |
|---|---|---|---|---|---|---|
| 125 | 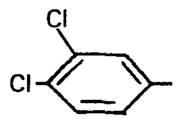 | H | H | O | $OCH_3$ | |
| 126 | do. | H | H | O | OH | |

Die Verbindungen werden angewendet, indem man die Pflanzen oder den Boden mit den Wirkstoffen besprüht oder bestäubt.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmässige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine oder Amide (z.B. Ethanolamin, Dimethylformamid) und Wasser; Trägerstoffe wie natürliche Gesteinsmehle, z.B. Kaoline, Tonerden, Talkum, Kreide und synthetische Gesteinsmehle (z.B. hochdisperse Kieselsäure, Silikate); Emulgiermittel, wie nichtionogene und anionische Emulgatoren (z.B. Polyoxyethylen – Fettalkohole – Ether, Alkylsulfonate und Arylsulfonate) und Dispergiermittel, wie Lignin, Sulfitablaugen und Methylcellulose.

Die herbiziden Mittel enthalten 0,1 bis 95 Gew.-% Wirkstoff, vorzugsweise 0,5 bis 90 Gew.-%.

Die Mittel bzw. die daraus hergestellten gebrauchsfertigen Zubereitungen, wie Lösungen, Emulsionen, Suspension, Pulver, Stäube, Pasten oder Granulate werden in bekannter Weise angewendet, beispielsweise durch Versprühen, Vernebeln, Verstäuben, Verstreuen oder Giessen.

Beispiele für solche Zubereitungen sind:

Beispiel I

Man vermischt 90 Gewichtsteile der Verbindung 1 mit 10 Gewichtsteilen N-Methyl-alpha-pyrrolidon und erhält eine Lösung, die zur Anwendung in Form kleinster Tropfen geeignet ist.

Beispiel II

10 Gewichtsteile der Verbindung 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht.

Beispiel III

20 Gewichtsteile der Verbindung 2 werden in einer Mischung gelöst, die aus 60 Gewichtsteilen Cyclohexanon, 30 Gewichtsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

Beispiel IV

20 Gewichtsteile der Verbindung 3 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht.

Beispiel V

80 Gewichtsteile des Wirkstoffs 1 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

Beispiel VI

5 Gewichtsteile der Verbindung 1 werden mit 95 Gewichtsteilen feinteiligem Kaolin innig vermischt. Man erhält auf diese Weise ein Stäubemittel, das 95 Gewichtsprozent des Wirkstoffs enthält.

Beispiel VII

30 Gewichtsprozent der Verbindung 1 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel VIII

40 Gewichtsteile des Wirkstoffs 1 werden mit 10 Teilen Natriumsalz eines Phenolsulfonsäure-harnstoff-formaldehyd-kondensats, 2 Teilen Kieselgel und 48 Teilen Wasser innig vermischt. Man erhält eine stabile wässrige Dispersion.

Beispiel IX

20 Teile des Wirkstoffs 1 werden mit 12 Teilen Calciumsalz der Dodecylbenzolsulfonsäure, 8 Teile Fettalkohol-polyglykolether, 2 Teilen Natriumsalz eines Phenolsulfonsäure-harnstoff-formaldehyd-kondensats und 68 Teilen eines paraffinischen Minieralöls innig vermischt. Man erhält eine stabile ölige Dispersion.

Die Wirkung der neuen Fluoralkancarbonsäureverbindungen auf das Wachstum von erwünschten und unerwünschten Pflanzen wird anhand von Gewächshausversuchen vorgeführt:

Als Kulturgefässe dienten Plastikblumentöpfe mit 300 $cm^3$ Inhalt und lehmigem Sand mit etwa 1,5% Humus als Substrat. Die Samen der Testpflanzen nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Bei dieser Applikationsmethode handelt es sich um eine Aufwandmenge entsprechend 3,0 kg Gesamtwirkstoff/ha. Nach dem Aufbringen der Mittel wurden die Gefässe leicht beregnet, um Keimung und Wachstum in Gang zu bringen. Danach deckte man die Gefässe mit durchsichtigen Plastikhauben ab, bis die Pflanzen angewachsen waren. Diese Abdeckung bewirkte ein gleichmässiges Keimen der Testpflanzen, sofern dies nicht durch die Wirkstoffe beeinträchtigt wurde. Zum Zwecke der Nachauflaufbehandlung zog man die Testpflanzen je nach Wuchsform erst bis zu einer Wuchshöhe von 5 bis 20 cm an und behandelte sie danach. Zur Nachauflaufbehandlung wurden entweder direkt gesäte und in den gleichen Gefässen aufgewachsene Pflanzen ausgewählt, oder sie wurden erst als Keimpflanzen getrennt angezogen und einige Tage vor der Behandlung in die Versuchsgefässe verpflanzt. Die Aufwandmengen für die Nachauflaufbehandlung waren bei einigen Verbindungen ebenfalls 3,0 kg Gesamtwirkstoff/ha. Weitere Beispiele wurden mit 1,0 kg/ha und 2,0 kg/ha Gesamtwirkstoff eingesetzt. Als Vergleichsmittel dienten das Dimethylaminsalz der 2,4-Dichlorphenoxy-a-propionsäure (A) mit 2,0 kg/ha berechnet als Säure und das Dimethylaminsalz der 2-Methyl-4-chlor-phenoxy-α-propionsäure (B) mit 1,0 kg/ha berechnet als Säure. Eine Abdeckung unterblieb bei der Nachauflaufbehandlung. Die Aufstellung der Versuche erfolgte im Gewächshaus, wobei für wärmeliebende Arten wärmere Bereiche (20 bis 30 °C) und für solche gemässigter Klimate 15 bis 25 °C bevorzugt wurden. Die Versuchsperiode erstreckte sich über 3 bis 4 Wochen.

Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die Ergebnisse zeigen, dass die Verbindungen eine beachtliche Wirkung bei Vorauflaufanwendung entfalten. Bei der vorzugsweisen Nachauflaufanwendung liegt die herbizide Aktivität ähnlich, bei einzelnen unerwünschten Pflanzen besser als bei den bekannten Vergleichsmitteln. Besonders hervorzuheben ist die günstigere Verträglichkeit der neuen Verbindungen für Kulturpflanzen.

Sind gewisse Kulturpflanzen gegenüber den Wirkstoffen etwas empfindlich, so können auch Ausbringungstechniken angewandt werden, bei welchen die herbiziden Mittel mit Hilfe der Spritzgeräte so geleitet werden, dass die Blätter empfindlicher Kulturpflanzen nach Möglichkeit nicht getroffen werden, während sie auf die Blätter darunter wachsender unerwünschter Pflanzen oder die unbedeckte Bodenfläche gelangen (postdirected, lay-by). In Anbetracht der guten Verträglichkeit und der Vielseitigkeit der Applikationsmethoden können die erfindungsgemässen Herbizide noch in einer weiteren grossen Zahl von Kulturpflanzen zur Beseitigung unerwünschten Pflanzenwuchses eingesetzt werden. Die Aufwandmengen können dabei zwischen 0,1 bis 15 kg/ha und mehr schwanken.

In Betracht kommen beispielsweise folgende Kulturen:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuss | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fodder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weisse Rübe | turnips |
| Brassica rapa var. silvestris | Rübsen | |
| Camellia sinensis | Teestrauch | tea plants |
| Carthamus tinctorius | Saflor-Färbedistel | safflower |
| Cary illinoinensis | Pekannussbaum | pecan trees |
| Citrus limon | Zitrone | lemon |
| Citrus maxima | Pampelmuse | grapefruits |

| Botanischer Name | Deutscher Name | Englischer Name |
|---|---|---|
| Citrus reticulata | Mandarine | |
| Citrus sinensis | Apfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawn |
| Daucus carota | Möhre | carrots |
| Elaeis guineensis | Ölpalme | oil palms |
| Fragaria vesca | Erdbeere | strawberries |
| Glyoine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakatuschukbaum | rubber plants |
| Hordeum vulgare | Gerste | barley |
| Humulus lupulus | Hopfen | hop |
| Ipomoea batatas | Süsskartoffeln | sweet potato |
| Juglans regia | Walnussbaum | walnut trees |
| Lactua sativa | Kopfsalat | lettuce |
| Lens culinaris | Linse | lentils |
| Linum usitatissimum | Faserlein | flax |
| Lycopersicon lycopersicum | Tomate | tomato |
| Malus spp. | Apfel | apple trees |
| Manihot esculenta | Maniok | cassava |
| Medicago sativa | Luzerne | alfalfa (lucerne) |
| Metha piperita | Pfefferminze | peppermint |
| Musa spp. | Obst- und Mehlbanane | banana plants |
| Nicotiana tabacum (N. rustica) | Tabak | tabacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Urdbohne | mungbeans |
| Phaseolus vulgaris | Buschbohnen | snapbeans, green beans, dry beans |
| Pennisetum glaucum | Perl- oder Rohrkolben-hirse | |
| Petroselinum crispum spp.tuberosum | Wurzelpetersilie | parsley |
| Picea abies | Rotfichte | Norway spruce |
| Abies alba | Weisstanne | fire |
| Pinus spp. | Kiefer | pine trees |
| Pisum sativum | Gartenerbse | English peas |
| Prunus avium | Süsskirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sasamum indicum | Sesam | Sesame |
| Solanum tuberosum | Kartoffel | Irish potatoes |
| Sorghum bicolor (s. vulgare) | Mohrenhirse | sorghum |
| Sorghum dochna | Zuckerhirse | |
| Spinacia oleracea | Spinat | spinach |
| Theobroma cacao | Kakaobaum | cacao plants |

| Botanischer Name | Deutscher Name | Englischer Name |
|---|---|---|
| Trifolium pratense | Rotklee | red clover |
| Triticum aestivum | Weizen | wheat |
| Vaccinium corymbosum | Kulturheidelbeere | blueberry |
| Vaccinium vitis-idaea | Preisselbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn maize |

Zur Verbreiterung des Wirkungsspektrums und zur Erzielung synergistischer Effekte können die neuen Fluoralkancarbonsäureverbindungen sowohl unter sich als auch mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht. Eine Reihe von Wirkstoffen, welche zusammen mit den neuen Verbindungen für verschiedenste Anwendungsbereiche sinnvolle Mischungen ergeben, werden beispielhaft aufgeführt:

α-Naphthoxyessigsäuremethylester
2-(2-Methylphenoxy)-propionsäure (Salze, Ester, Amide)
2-(4-Chlorphenoxy)-propionsäure (Salze, Ester, Amide)
2-(2,4-Dichlorphenoxy)-propionsäure (Salze, Ester, Amide)
2-(2,4,5-Trichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
Cyclohexyl-3-(2,4-dichlorphenoxy)-acrylat
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure (Salze, Ester)
Gibellerinsäure (Salze)
Dinatrium-methylarsonat
Mononatriumsalz der Methylarsonsäure
N-Phosphon-methyl-glycin (Salze)
N,N-Bis-(phosphonmethyl)-glycin (Salze)
2-Chlorethanphosphonsäure-2-chlorethylester
Ammonium-ethyl-carbamoyl-phosphonat
Di-n-butyl-1-n-butylamino-cyclohexyl-phosphonat
Trithiobutylphosphit
O,O-Diisopropyl-5-(2-benzosulfonylamino-ethyl)-phosphordithioat
2,3-Dihydro-5,6-dimethyl-1,4-dithiin-1,1,4,4-tetraoxid
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bernsteinsäure-mono-N-dimethylhydrazid (Salze)
(2-Chlorethyl)-trimethyl-ammoniumchlorid
(2-Methyl-4-phenylsulfonyl)-trifluormethansulfonanilid
1,1-Dimethyl-4,6-diisopropyl-5-indanylethylketon
Natriumchlorat
Ammoniumrhodanid
Calciumcyanamid
5-Amino-4-chlor-2-phenyl-3(2H)-pyridazinon
5-Amino-4-brom-2-phenyl-3(2H)-pyridazinon
5-Amino-4-chlor-2-cyclohexyl-3(2H)-pyridazinon
5-Amino-4-brom-2-cyclohexyl-3(2H)-pyridazinon
5-Methylamino-4-chlor-2-(3.trifluormethylphenyl-3(2H)-pyridazinon
5-Methylamino-4-chlor-2-(3,α,α,β,β-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
4,5-Dimethoxy-2-cyclohexyl-3(2H)-pyridazinon
4,5-Dimethoxy-2-(3.trifluormethylphenyl)-3(2H)-pyridazinon
5-Methoxy-4-chlor-2-(3.trifluormethylphenyl)-3(2H)-pyridazinon
5-Amino-4-brom-2-(3.methylphenyl)-3(2H)-pyridazinon
3-(1-Methylethyl)-1H-2,1,3-benzothiadiazon-4(3H)-on-2,2- dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze
1-Methoxymethyl-3-(1-methylethyl-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4-(3H)-on-2,2-dioxid
1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Cyan-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-pyridino-[3,2-e]2,1,3-thiadiazin-(4)-on-2,2-dioxid
N-(1-Ethylpropyl)-2,6-dinitro-3,4-dimethylanilin
N-(1-Methyläthyl)-N-ethyl-2,6-dinitro-4-trifluormethylanilin

N-n.Propyl-N-b-chlorethyl-2,6-dinitro-4-trifluormethylanilin
N-n.Propyl-N-cyclopropylmethyl-2,6-dinitro-4-trifluormethyl-anilin
N-Bis-(n.propyl)-2,6-dinitro-4-amino-4-trifluormethylanilin
N-Bis-(n.propyl)-2,6-dinitro-4-methyl-anilin
N-Bis-(n.propyl)-2,6-dinitro-4-methylsulfonyl-anilin
N-Bis-(n.propyl)-2,6-dinitro-4-aminosulfonyl-anilin
Bis-(β-chlorethyl)-2,6-dinitro-4-methyl-anilin
N-Ethyl-N-(2-methylallyl)-2,6-dinitro-4-trifluormethylanilin
N-Methylcarbaminsäure-3,4-dichlorbenzylester
N-Methylcarbaminsäure-2,6-di-tert.butyl-4-methylphenylester
N-Phenylcarbaminsäure-isopropylester
N-3-Fluorphenylcarbaminsäure-3-methoxypropyl-2-ester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-1-ester
N-3,4-Dichlorphenylcarbaminsäure-methylester
N-(4-Amino-benzolsulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionsäureanilid
Ethyl-N-(3-(N'-phenylcarbamoyloxy)-phenyl)-carbamat

Methyl-N-(3-(N'-methyl-N'-phenylcarbamoyloxy)-phenyl)-carbamat
Isopropyl-N-(3-(N'-ethyl-N'-phenylcarbamoyloxy)-phenyl)-carbonat
Methyl-N-(3-(N'-3-methylphenylcarbamoyloxy)-phenyl)-carbamat
Methyl-N-(3-(N'-4-fluorphenylcarbamoyloxy)-phenyl)-carbamat
Methyl-N-(3-(N'-3-chlor-4-fluorphenylcarbamoyloxy)-phenyl)-carbamat
Ethyl-N-(3-(N'-3-chlor-4-fluorphenylcarbamoyloxy)-phenyl)-carbamat
Ethyl-N-(3-(N'-3,4-difluorphenylcarbamoyloxy)-phenyl)- carbamat
Methyl-N-(3-(N'-3,4-difluorphenylcarbamoyloxy)-phenyl)-carbamat
N-3-(4-Fluorphenoxycarbonylamino)-phenylcarbaminsäuremethylester
N-3-(2-Methylphenoxycarbonylamino)-phenylcarbaminsäuremethylester
N-3-(4-Fluorphenoxycarbonylamino)-phenylthiolcarbaminsäuremethylester
N-3-(2,4,5-Trimethylphenoxycarbonylamino)-phenylthiolcarbaminsäure-methylester
N-3-(Phenoxycarbonylamino)-phenylthiolcarbaminsäuremethylester
N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolylmethylester
N,N-Di-isopropyl-thiolcarbaminsäure-3-ethyl-5-isoxazolylmethylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo-[2,2,1]-heptyl-thiolcarbaminsäureethylester
S-(2,3-Dichlorallyl)-(2,2,4-trimethyl-azetidin)-1-carbothiolat
S-(2,3,3-Trichlorallyl)-(2,2,4-trimethyl-azetidin)-1-carbothiolat
S-Ethyl-hexahydro-1-H-azepin-1-carbothiolat
S-Benzyl-3-methylhexahydro-1-H-azepin-1-carbothiolat
S-Benzyl-2,3-dimethylhexahydro-1-H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
α,α-Dichlorpropionsäure-Natriumsalz
α,α-Dichlorbuttersäure-Natriumsalz
α,α, β,β-Tetrafluorpropionsäure-Natriumsalz
α-Methyl,α,β-dichlorpropionsäure-Natriumsalz
α-Chlor-β-(4-chlorphenyl)-propionsäure-methylester
α,β-Dichlor-β-phenylpropionsäure-methylester
Benzamido-oxy-essigsäure
2,3,5-Triiodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)
O,S-Dimethyltetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäure-isobutylester
3-[4-(2', 4'-Dichlorphenoxy)-phenoxy]-propionsäuremethylester
2-[4-(4'-Trifluormethylphenoxy)-phenoxy]-propionsäuremethyl-ester
2-[4-(2'-Chlor-4'-trifluorphenoxy)-phenoxy]-propionsäure-Natriumsalz
2-[4-(3', 5'-Dichlorpyridyl-2-oxy)-phenoxy]-propionsäure-Natriumsalz

2-(N-Benzoyl-3,4-dichlorphenylamino)-propionsäureethylester
2-(N-Benzoyl-3-chlor-4-fluorphenylamino)-propionsäuremethylester
2-(N-Benzoyl-3-chlor-4-fluorphenylamino)-propionsäure-isopropylester
2-Chlor-4-ethylamino-6-isopropylamino-1,3,5-triazin
2-Chlor-4-ethylamino-6-(amino-2'-propionitril)-1,3,5-triazin
2-Chlor-4-ethylamino-6-2-methoxypropyl-2-amino-1,3, 5-triazin
2-Chlor-4-ethylamino-6-butin-1-yl-2-amino-1,3,5-triazin
2-Chlor-4,6-bisethylamino-1,3,5-triazin
2-Chlor-4,6-bisisopropylamino-1,3, 5-triazin
2-Chlor-4-isopropylamino-6-cyclopropylamino-1,3,5-triazin
2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5 –triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4-dihydro-1,2,4-triazin-5-on
1-Methyl-3-cyclohexyl-6-dimethylamino-1,3,5-triazin-2,4-dion
3-tert.Butyl-5-chlor-6-methyluracil
3-tert.Butyl-5-brom-6-methyluracil
3-Isopropyl-5-brom-6-methyluracil
3-sec.Butyl-5-brom-6-methyluracil
3-(2-Tetrahydropyranyl)-5-chlor-6-methyluracil
3-(2-Tetrahydropyranyl)-5,6-trimethylenuracil
3-Cyclohexyl-5,6 –trimethyluracil
2-Methyl-4-(3'-trifluormethylphenyl)-tetrahydro-1,2, 4-oxadiazin-3,5-dion
2-Methyl-4-(4'-fluorphenyl)-tetrahydro-1,2,4-oxadiazin-3,5-dion
3-Amino-1,2,4-triazol
1-Allyloxy-1-(4-bromphenyl) -2-[1', 2', 4'-triazolyl-(1')-]-ethan (Salze)
1-(4-Chlorphenoxy- 3,3-dimethyl-1(1H-1,2,4-triazol-1-yl)-2-butanon
N,N-Diallylchloracetamid
N-Isopropyl-2-chloracetanilid
N-(1-Methyl-propin-2-yl)-2-chloracetanilid
2-Methyl-6-ethyl-N-(propargyl)-2-chloracetanilid
2-Methyl-6-ethyl-N-(ethoxymethyl)-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracetanilid
2-Methyl-6-ethyl-N-(4-methoxypyrazol-1-yl-methyl)-2-chloracetanilid
2-Methyl-6-ethyl-N-(pyrazol-1-yl -methyl)-2-chloracetanilid
2,6-Dimethyl-N-(pyrazol-1-yl-methyl)-2-chloracetanilid
2,6-Dimethyl-N-(4-methylpyrazol-1-yl-methyl)-2-chloracetanilid
2,6-Dimethyl-N-(1,2,4-triazol-1-yl-methyl)-2-chloracetanilid
2,6-Dimethyl-N-(3,5-dimethylpyrazol-1-yl-methyl)-2-chloracetanilid
2,6-Dimethyl-N-(1,3-dioxolan-2-yl-methyl)-2-chloracetanilid
2,6-Dimethyl-N-(2-methoxyethyl)-2-chloracetanilid
2,6-Dimethyl-N-(isobutoxymethyl)-2-chloracetanilid
2,6-Diethyl-N-(methoxymethyl-)-2-chloracetanilid
2,6-Diethyl-N-(n-butoxymethyl)-2-chloracetanilid
2,6-Diethyl-N-(ethoxycarbonylmethyl)-2-chloracetanilid
2,3,6-Trimethyl-N-(pyrazol-1-yl-methyl)-2-chloracetanilid
2,3-Dimethyl-N-(isopropyl)-2-chloracetanilid
2,6-Diethyl-N-(2-n-propoxyethyl)-2-chloracetanilid
2-(2-Methyl-4-chlorphenoxy)-N-methoxy-acetamid
2-(α-Naphtoxy)-N,N-diethylpropionamid
2,2-Diphenyl-N,N-dimethylacetamid
α-(3,4,5-Tribrompyrazol-1-yl)-N,N-dimethylpropionamid
N-(1,1-Dimethylpropinyl)-3,5-dichlorbenzamid
N-1-Naphthylphthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid
2-Propionyl-amino-4-methyl-5-chlor-thiazol
0-(Methylsulfonyl)-glykolsäure-N-ethoxymethyl-2,6-dimethylanilid
0-(Methylaminosulfonyl)-glykolsäure-N-isopropyl-anilid
0-(i-Propylaminosulfonyl)-glykolsäure-N-butin-1-yl-3-anilid
0-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Diiod-4-hydroxy-benzonitril (Salze)
3,5-Dibrom-4-hydroxy-0-2,4-dinitrophenylbenzaldoxim (Salze)
3,5-Dibrom-4-hydroxy-0-2-cyan-4-nitrophenylbenzaldoxim (Salze)
Pentachlorphenol-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether

2,4-Dichlorphenyl-3'-methoxy-4' -nitro-phenylether
2-Chlor-4-trifluormethylphenyl-3'-ethoxy-4'-nitrophenylether
2-Chlor-4-trifluormethylphenyl-3'-carboxy-4'-nitro-phenylether (Salze)
2,4-Dichlorphenyl-3'-methoxycarbonyl-4'-nitrophenylether
2-(3,4-Dichlorphenyl)-4-methyl-1,2,4-oxadiazolidin-3,5-dion
2-(3-tert.Butylcarbamoyl-oxyphenyl)-4-methyl-1,2,4-oxadiazolidin-3,5-dion
2-(3-iso-Propylcarbamoyl-oxyphenyl)-4-methyl-1,2,4-oxadiazolidin-3,5-dion
2-Phenyl-3,1-benzoxazinon-(4)
(4-Bromphenyl)-3,4,5,9,10-pentaazatetracyclo-[5,4,1,$0^{2,6}$0,$^{8,11}$]-dodeca-3,9-dien
2-Ethoxy-2,3-dihydro-3,3-dimethyl-5-benzofuranyl-methansulfonat
2-Ethoxy-2,3-dihydro-3,3-dimethyl-5-benzofuranyl-dimethylaminosulfonat
2-Ehtoxy-2,3-dihydro-3,3-dimethyl-5-benzofuranyl-(N-methyl-N-acetyl)-aminosulfonat
3,4-Dichlor-1,2-benzisothiazol
N-4-Chlorphenyl -allylbernsteinsäureamid
2-Methyl-4,6-dinitrophenol (Salze, Ester)
2-sec.Butyl-4,6-dinitrophenol (Salze)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(α,α-Dimethylbenzyl)-3-(4-methylphenyl)-harnstoff
1-Phenyl-3-(2-methylcyclohexyl)-harnstoff
1-Phenyl-1-benzoyl-3,3-dimethyl-harnstoff
1-(4-Chlorphenyl)-1-benzoyl-3,3-dimethyl-harnstoff
1-(4-Chlorphenyl)-3,3-dimethyl-harnstoff
1-(4-Chlorphenyl)-3-methyl-3-butin-1-yl-3-harnstoff
1-(3,4-Dichlorphenyl)-3,3-dimethyl-harnstoff
1-(3,4-Dichlorphenyl)-1-benzoyl-3,3-dimethyl-harnstoff
1-(3,4,Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(3-α, α,β,β-Tetrafluorethoxyphenyl)-3,3-dimethyl-harnstoff
1-(3-tert. -Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-(3,5-Dichlor-4-methoxyphenyl)-3,3-dimethyl-harnstoff
1-[4(4'-Chlorphenoxy)-phenyl]-3,3-dimethyl-harnstoff
1-[4 (4'-methoxyphenoxy)-phenyl]-3,5-dimethyl-harnstoff
1-Cyclooctyl-3,5-dimethyl-harnstoff
1-(Hexahydro-4,7-methanindan-5-yl)-3,3-dimethyl-harnstoff
1-[1- oder 2-(3a, 4,5,7,7a-Hexahydro)-4,7-methanoindanyl]-3,3-dimethyl-harnstoff
1-(4-Fluorphenyl)-3-carboxymethoxy-3-methyl-harnstoff
1-Phenyl-3-methyl-3-methoxy-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff
1-(3-Chlor-4-isopropylphenyl)-3-methyl-3-methoxy-harnstoff
1-(3-Chlor-4-methoxyphenyl)-3-methyl-harnstoff
1-(3-tert.Butxlphenyl)-3-methyl-3-methoxy-harnstoff
1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethylharnstoff
Imidazolidin-2-on-1-carbonsäure-iso-butylamid
1,2,-Dimethyl-3,5-diphenylpyrazolium-methylsulfat
1,2,4-Trimethyl-3,5-diphenylpyrazolium-methylsulfat
1,2-Dimethyl-4-brom-3,5-diphenylpyrazolium-methylsulfat
1,3-Dimethyl-4-(3,4-dichlorbenzoyl)-5-[4-methylphenyl)-sulfonyl-oxyl]-pyrazol
2,3,5-Trichlor-pyridinol-(4)
1-Methyl-3-phenyl-5-(3'-trifluormethylphenyl)-pyridon-(4)
1-Methyl-4-phenyl-pyridiniumchlorid
1,1-Dimethylpyridiniumchlorid
3-Phenyl-4-hydroxy-6-chlorpyridazin
1,1'-Dimethyl-4,4'-dipyridylium-di-(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Alllyoxyamino)-propyliden]-5,5-dimethylcyclohexan-1,3-dion (Salze)
2-[1-(N-Allyloxyamino)-butyliden]-5,5-dimethylcyclohexan-1,3-dion (Salze)
2-[1-(N-Allyloxyamino)-butyliden]-5,5-dimethyl-4-methoxycarbonyl-cyclohexyn-1,3-dion (Salze)
2-Chlorphenoxyessigsäure (Salze, Ester, Amide)
4-Chlorphenoxyessigsäure (Salze, Ester, Amide)
2,4-Dichlorphenoxyessigsäure (Salze, Ester, Amide)
2,4,5-Trichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)
2-Chlor-4-trifluormethyl-3'-ethoxycarbonyl-4'-nitrophenylether
1-(4-Benzyloxyphenyl)-3-methyl-3-methoxyharnstoff
2-[1-(2,5-Dimethylphenyl)-ethylsulfonyl]-pyridin-N-oxid

1-Acetyl-3-anilino-4-methoxycarbonyl-5-methyl-
pyrazol
3-Anilino-4-methoxycarbonyl-5-methylpyrazol
3-tert.Butylamino-4-methoxycarbonyl-5-methyl-
pyrazol
N-Benzyl-N-isopropyl-trimethylacetamid
2-[4-(4'-Chlorphenoxymethyl)-phenoxy]-pro-
pionsäuremethylester
2-[4-(5'-Brompyridyl-2-oxy)-phenoxy]-propion-
säuremethylester
2-[4-(5'-Jodpyridyl-2-oxy)-phenoxy]-propionsäu-
remethylester
2-[4-(5'-Jodpyridyl-2-oxy)-phenoxy]-propionsäure-n.-butylester
2-Chlor-4-trifluormethylphenyl-3'-(2-fluorethoxy)-
4'-nitrophenylether
2-Chlor-4-trifluormethylphenyl-3-(ethoxycarbonyl)methylthio-4-nitrophenylether
2,4,6-Trichlorphenyl-3(ethoxycarbonyl)methyl-
thio-4-nitrophenylether
2-[1-(N-ethoxamino)-butyliden]-5-(2-ethylthiopro-
pyl)-3-hydroxy-cyclohexen-(2)-on(1) (Salze)
2-[1-(N-ethoxamino)-butyliden]-5-(2-phenylthio-
propyl)-3-hydroxy-cyclohexen-(2)-on-(1) (Salze)
4-[4-(4'-Trifluormethyl)-phenoxy]-penten-2-
carbonsäureethylester
2-Chlor-4-trifluormethyl-3'-methoxycarbonyl-4'-
nitrophenylther
2,4-Dichlorphenyl-3'-carboxy-4'-nitrophenylether
(Salze)
4,5-Dimethoxy-2-(3-α,α,β-trifluor-β-brometh-
oxyphenyl)-3-(2H)-pyridazinon
2,4-Dichlorphenyl-3'-ethoxy-ethoxy-ethoxy-4'-ni-
trophenylether
2,3-Dihydro-3,3-dimethyl-5-benzofuranyl-ethan-
sulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethyl-
ester
2-Chlor-3,5-dijod-4-acetoxy-piridin
1-(4-[2-(4-Methylphenyl)-äthoxy]-phenyl-3-methyl-3-methoxyharnstoff
4,5-Dimethoxy-2-(3-α,α,β-trifluor-β-brometh-
oxyphenyl)-3-(2H)-pyridazinon
2,4-Dichlorphenyl-3'-ethoxy-ethoxy-ethoxy-4'-ni-
trophenylether
2,3-Dihydro-3,3-dimethyl-5-benzofuranyl-ethan-
sulfonat
N-[4-Methoxy-6-methyl-1,3,5-triazin-2-yl-aminocarbonyl]-2-chlorbenzolsulfonamid
1(3-Chlor-4-ethoxyphenyl)-3,3-dimethylharnstoff
2-Methyl-4-Chlorphenoxy-thioessigsäureethyl-
ester
2-Chlor-3,5-dijod-4-acetoxy-piridin
1-(4-[2-(4-Methylphenyl)-äthoxy]-phenyl)-3-me-
thyl-3-methoxyharnstoff.

Ausserdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien.Von Interesse ist ferner die Mischbarkeit mit Mineralsalzlösungen, welche zur Behebung von Ernährungs- oder Spurenelementmängeln eingesetzt werden. Es können auch nichtphytotoxische Öle und Ölkonzentrate zugesetzt werden.

Bei der Prüfung auf selektive herbizide Wirkung bei Nachauflaufanwendung im Gewächshaus bei Aufwandmengen von 2,0 kg Wirkstoff/ha zeigt die neue Verbindung 2 eine bessere selektive herbizide Wirkung als der bekannte Wirkstoff A.

Ebenso hat der Wirkstoff Nr. 3 bei der selektiven Unkrautbekämpfung bei Nachauflaufanwendung im Gewächshaus bei Aufwandmengen von 1,0 kg Gesamtwirkstoff/ha eine bessere selektive herbizide Wirkung als der bekannte Wirkstoff B in derselben Aufwandmenge berechnet auf Säurebasis.

Bei der Prüfung der herbiziden Wirkung bei Vor- und Nachauflaufbehandlung im Gewächshaus bei Aufwandmengen von 3 kg/ha Gesamtwirkstoff zeigen die neuen Wirkstoffe Nr. 6, 8, 11, 22 und 56 eine recht gute herbizide Aktivität.

In weiteren Versuchen bei Nachauflaufanwendung im Gewächshaus zeigten die Verbindungen Nr. 27 und Nr. 25 bei 1,0 kg/ha eine sehr gute bzw. akzeptable Verträglichkeit für die Kulturpflanze Reis. Gleichzeitig wurden breitblättrige unerwünschte Pflanzen empfindlich geschädigt. Entscheidend hierfür ist die sehr gute Verträglichkeit der Verbindungen für eine Kulturpflanze aus der Familie der Gramineen.

Tabelle 1 Liste der Pflanzennamen

| Botanischer Name | Abkürzung in Tabelle | Deutscher Name | Englischer Name |
|---|---|---|---|
| Arachys hypogaea | Arachys hyp. | – | peanuts |
| Chenopodium album | – | Weisser Gänsefuss | lambsquarters |
| Galium aparine | – | Klebkraut, Kletten-Laubkraut | catchweed bedstraw |
| Hordeum vulgare | Hordeum vulg. | Gerste | barley |
| Ipomoea spp. | – | Prunkwindearten | morningglory |
| Sinapis alba | – | Weisser Senf | white mustard |
| Triticum aestivum | Tritic. aest. | Weizen | wheat |
| Amaranthus retroflexus | – | zurückgekrümmter Fuchsschwanz | redroot pigweed |
| Matricaria spp. | – | Kamillearten | chamomile |
| Oryza sativa | – | Reis | rice |

**Patentansprüche für die Vertragsstaaten: BE, CH, DE, FR, GB, IT, LI, LU, NL, SE**

1. 2-Fluoralkancarbonsäure-Verbindung der allgemeinen Formel

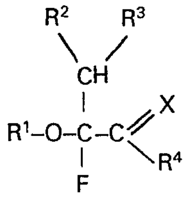

worin

$R^1$ einen gegebenenfalls durch Halogen, Nitro, einen geradkettigen Alkylrest mit 1 bis 14, einen verzweigten oder cyclischen Alkylrest mit 3 bis 14 Kohlenstoffatomen, einen Alkoxy- oder Alkylthiorest mit 1 bis 5 Kohlenstoffatomen, einen Halogenalkyl-, Halogenalkoxy-, Halogenalkylthio- oder Halogenalkylsulfonylrest mit bis zu 4 Kohlenstoffatomen und bis zu 9 Halogenatomen, eine Formyl-, Cyano- oder Thiocyanatgruppe, einen Alkoxycarbonyl-, Dialkylketo-, Alkylcarbonyl-, Alkyl- und Dialkylcarbamoyl-, Acylamino- oder einen Alkylaminosulfonylrest mit 2 bis 10 Kohlenstoffatomen, einen Alkoxyalkyl- oder Alkylthioalkylrest mit 2 bis 6 Kohlenstoffatomen, einen Phenyl- oder Phenylcarbonylrest mit 6 bis 11 Kohlenstoffatomen, einen gegebenenfalls durch Chlor oder Trifluormethyl substituierten Aryloxy-, Arylalkyloxy- oder Arylazorest, Arylthio-, Arylsulfinyl- oder Arylsulfonylrest, einen Arylalkylrest, einen geradkettigen Alkenylrest mit 2 bis 6 oder einen verzweigten Alkenylrest mit 3 bis 7 Kohlenstoffatomen, einen geradkettigen Alkinylrest mit 2 bis 4 oder einen verzweigten Alkinylrest mit 3 bis 6 Kohlenstoffatomen, einen ankondensierten Benzo-, Benzofuran- oder Pyridinorest substituierten Aryl- oder Heteroarylrest,

$R^2$ und $R^3$ unabhängig voneinander Wasserstoff, einen gegebenenfalls wie bei $R^1$ substituierten Aryl- oder Heteroarylrest oder einen gegebenenfalls durch Chlor, Brom, Fluor, Nitro, Cyan, Methoxy oder Ethoxy substituierten Alkylrest oder einen gegebenenfalls durch Chlor oder Trifluormethyl substituierten Cycloalkylrest oder einen Aralkylrest,

X Sauerstoff oder Schwefel und

$R^4$ eine Hydroxylgruppe oder die Gruppen $OR^5$, $SR^5$ oder

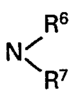 bedeuten, worin

$R^5$ einen gegebenenfalls wie bei $R^1$ substituierten Aryl- oder Heteroarylrest oder einen gegebenenfalls durch Chlor, Fluor, Brom, Methoxy, Ethoxy, Butoxy, Cyclohexoxy, Methylmercapto oder Ethylmercapto substituierten Alkylrest, einen gegebenenfalls durch Chlor substituierten Alkenylrest, einen gegebenenfalls durch Chlor substituierten Alkinylrest, einen Cycloalkylrest, einen Cycloalkenylrest, einen Alkanonrest oder einen Heteroarylalkylrest,

$R^6$ Wasserstoff oder die für $R^5$ genannten Reste, ausser einem gegebenenfalls substituierten m-Anilinorest,

$R^7$ unabhängig von $R^6$ die für $R^5$ genannten Reste oder eine Hydroxylgruppe oder einen Alkoxy- oder Alkylthiorest, eine Cyano- oder Cyanoalkylgruppe oder den Rest

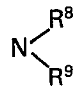 bedeutet,

worin $R^8$ und $R^9$ unabhängig voneinander Wasserstoff oder einen gegebenenfalls wie bei $R^1$ substituierten Arylrest oder einen Alkylrest bedeuten und ferner $R^6$ und $R^7$ zusammen mit dem benachbarten Stickstoffatom einen gegebenenfalls durch Methyl substituierten heterocyclischen Rest bedeuten, der gegebenenfalls eine oder mehrere Doppelbindungen enthält oder zusätzlich zu dem Stickstoffatom ein oder mehrere Heteroatome enthält, oder die Salze dieser Verbindung.

2. Herbizid, enthaltend eine 2-Fluoralkancarbonsäure-Verbindung gemäss Anspruch 1.

3. Herbizid, enthaltend einen festen oder flüssigen Trägerstoff und eine 2-Fluoralkancarbonsäure-Verbindung gemäss Anspruch 1.

4. Verfahren zur Herstellung eines Herbizids, dadurch gekennzeichnet, dass man einen festen oder flüssigen Trägerstoff vermischt mit einer 2-Fluoralkancarbonsäure-Verbindung gemäss Anspruch 1.

5. Verfahren zur Bekämpfung von unerwünschten Pflanzen, dadurch gekennzeichnet, dass man die Pflanzen oder den Boden behandelt mit einer 2-Fluoralkancarbonsäure-Verbindung gemäss Anspruch 1.

6. Verfahren zur Herstellung einer 2-Fluoralkancarbonsäureverbindung gemäss Anspruch 1, dadurch gekennzeichnet, dass man

a) eine Acrylsäureverbindung der allgemeinen Formel

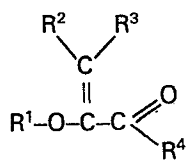 II

worin $R^1$, $R^2$, $R^3$ und $R^4$ die im Anspruch 1 genannten Bedeutungen haben, mit Flusssäure mit oder ohne Verdünnungsmittel oder einem Katalysator umsetzt und die so erhaltenen Verbindungen gegebenenfalls schwefelt, oder

b) eine 2-Fluoralkancarbonsäure der Formel

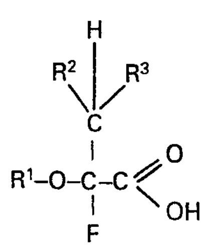

worin $R^1$, $R^2$ und $R^3$ die im Anspruch 1 genannten Bedeutungen haben oder deren Salze mit Thionylchlorid, Phosgen, Phosphorpentachlorid, N,N'-Carbonyldiimidazol, N,N'-Carbonylditriazol, N,N'-Thionyldiimidazol oder N,N'-Thionyltriazol oder deren Mischung gegebenenfalls in Gegenwart eines Verdünnungsmittels umsetzt und danach mit einer Verbindung der Formel

$$HR^4$$

gegebenenfalls in Gegenwart eines Verdünnungsmittels, einer Base oder eines Reaktionsbeschleunigers umsetzt, wobei $R^4$ die im Anspruch 1 genannten Bedeutungen ausser der Hydroxylgruppe hat, und die so erhaltene Verbindung gegebenenfalls schwefelt.

7. 2-Fluoralkancarbonsäure-Verbindung gemäss Anspruch 1, nämlich 2-(2',4'-Dichlorphenoxy)-2-fluorpropionsäure, 2-(2',4'-Dichlorphenoxy)-2-fluorpropionsäuremethylester, 2-(2',4'-Dichlorphenoxy)-2-fluorpropionsäureisooctylester, 2-(3'-Trifluormethylphenoxy)- 2-fluorpropionsäuremethylester, 2-(4'-Fluorphenoxy)-2-fluorpropionsäuremethylester, 2-(2'-Methyl-4'-chlorphenoxy)-2-fluorpropionsäure oder 2-(2'4'-Dichlorphenoxy)-2-fluorpropionsäure-2''-(3''-chlorphenoxy)-ethylester.

8. Herbizid gemäss Anspruch 2, enthaltend eine 2-Fluoralkancarbonsäure-Verbindung, nämlich 2-(2',4'-Dichlorphenoxy)- 2-fluorpropionsäure, 2-(2',4'-Dichlorphenoxy)-2-fluorpropionsäuremethylester, 2-(2',4'Dichlorphenoxy)-2-fluorpropionsäureisooctylester, 2-(3'-Trifluormethylphenoxy)-2-fluorpropionsäuremethylester, 2-(4'-Fluorphenoxy)-2-fluorpropionsäuremethylester, 2-(2'-Methyl-4'-chlorphenoxy)-2-fluorpropionsäure oder 2-(2',4'-Dichlorphenoxy)-2-fluorpropionsäure-2''-(3''-chlorphenoxy)-ethylester.

**Patentansprüche für den Vertragsstaat: AT**

1. Herbizid, enthaltend eine 2-Fluoralkancarbonsäure-Verbindung der allgemeinen Formel

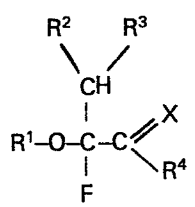

worin

$R^1$ einen gegebenenfalls durch Halogen, Nitro, einen geradkettigen Alkylrest mit 1 bis 14, einen verzweigten oder cyclischen Alkylrest mit 3 bis 14 Kohlenstoffatomen, einen Alkoxy- oder Alkylthiorest mit 1 bis 5 Kohlenstoffatomen, einen Halogenalkyl-, Halogenalkoxy-, Halogenalkylthio- oder Halogenalkylsulfonylrest mit bis zu 4 Kohlenstoffatomen und bis zu 9 Halogenatomen, eine Formyl-, Cyano- oder Thiocyanatgruppe, einen Alkoxycarbonyl-, Dialkylketo-, Alkylcarbonyl-, Alkyl- und Dialkylcarbamoyl-, Acylamino- oder einen Alkylaminosulfonylrest mit 2 bis 10 Kohlenstoffatomen, einen Alkoxyalkyl- oder Alkylthioalkylrest mit 2 bis 6 Kohlenstoffatomen, einen Phenyl- oder Phenylcarbonylrest mit 6 bis 11 Kohlenstoffatomen, einen gegebenenfalls durch Chlor oder Trifluormethyl substituierten Aryloxy-, Aryalkyloxy- oder Arylazorest, Arylthio-, Arylsulfinyl- oder Arylsulfonylrest, einen Arylalkylrest, einen geradkettigen Alkenylrest mit 2 bis 6 oder einen verzweigten Alkenylrest mit 3 bis 7 Kohlenstoffatomen, einen geradkettigen Alkinylrest mit 2 bis 4 oder einen verzweigten Alkinylrest mit 3 bis 6 Kohlenstoffatomen, einen ankondensierten Benzo-, Benzofuran- oder Pyridinorest substituierten Aryl- oder Heteroarylrest,

$R^2$ und $R^3$ unabhängig voneinander Wasserstoff, einen gegebenenfalls wie bei $R^1$ substituierten Aryl- oder Heteroarylrest oder einen gegebenenfalls durch Chlor, Brom, Fluor, Nitro, Cyan, Methoxy oder Ethoxy substituierten Alkylrest oder einen gegebenenfalls durch Chlor oder Trifluormethyl substituierten Cycloalkylrest oder einen Aralkylrest,

X Sauerstoff oder Schwefel und

$R^4$ eine Hydroxylgruppe oder die Gruppen $OR^5$, $SR^5$ oder

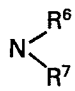 bedeuten, worin

$R^5$ einen gegebenenfalls wie bei $R^1$ substituierten Aryl- oder Heteroarylrest oder einen gegebenenfalls durch Chlor, Fluor, Brom, Methoxy, Ethoxy, Butoxy, Cyclohexoxy, Methylmercapto oder Ethylmercapto substituierten Alkylrest, einen gegebenenfalls durch Chlor substituierten Alkenylrest, einen gegebenenfalls durch Chlor substituierten Alkinylrest, einen Cycloalkylrest, einen Cycloalkenylrest, einen Alkanonrest oder einen Heteroarylalkylrest,

$R^6$ Wasserstoff oder die für $R^5$ genannten Reste, ausser einem gegebenenfalls substituierten m-Anilinorest,

$R^7$ unabhängig von $R^6$ die für $R^5$ genannten Reste oder eine Hydroxylgruppe oder einen Alkoxy- oder Alkylthiorest, eine Cyano- oder Cyanoalkylgruppe oder den Rest

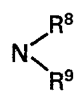 bedeutet,

worin $R^8$ und $R^9$ unabhängig voneinander Wasserstoff oder einen gegebenenfalls wie bei $R^1$ substituierten Arylrest oder einen Alkylrest bedeuten und ferner $R^6$ und $R^7$ zusammen mit dem benachbarten Stickstoffatom einen gegebenenfalls durch Methyl substituierten heterocyclischen Rest bedeuten, der gegebenenfalls eine oder mehrere Doppelbindungen enthält oder zusätzlich zu dem Stickstoffatom ein oder mehrere Heteroatome enthält, oder die Salze dieser Verbindung.

2. Herbizid, enthaltend einen festen oder flüssigen Trägerstoff und eine 2-Fluoralkancarbonsäure-Verbindung definiert wie im Anspruch 1.

3. Verfahren zur Herstellung eines Herbizids, dadurch gekennzeichnet, dass man einen festen

oder flüssigen Trägerstoff vermischt mit einer 2-Fluoralkancarbonsäure-Verbindung definiert wie im Anspruch 1.

4. Verfahren zur Bekämpfung von unerwünschten Pflanzen, dadurch gekennzeichnet, dass man die Pflanzen oder den Boden behandelt mit einer 2-Fluoralkancarbonsäure-Verbindung definiert wie im Anspruch 1.

5. Verfahren zur Herstellung einer 2-Fluoralkancarbonsäureverbindung definiert wie im Anspruch 1, dadurch gekennzeichnet, dass man

a) eine Acrylsäureverbindung der allgemeinen Formel

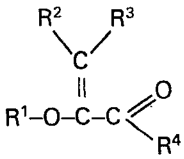

II

worin $R^1$, $R^2$, $R^3$ und $R^4$ die im Anspruch 1 genannten Bedeutungen haben, mit Flusssäure mit oder ohne Verdünnungsmittel oder einem Katalysator umsetzt und die so erhaltenen Verbindungen gegebenenfalls schwefelt, oder

b) eine 2-Fluoralkancarbonsäure der Formel

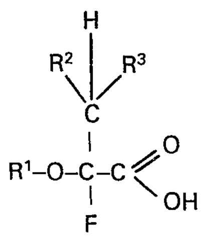

worin $R^1$, $R^2$ und $R^3$ die im Anspruch 1 genannten Bedeutungen haben oder deren Salze mit Thionylchlorid, Phosgen, Phosphorpentachlorid, N,N'-Carbonyldiimidazol, N,N'-Carbonylditriazol, N,N'-Thionyldiimidazol oder N,N'-Thionyltriazol oder deren Mischung gegebenenfalls in Gegenwart eines Verdünnungsmittels umsetzt und danach mit einer Verbindung der Formel

$$HR^4$$

gegebenenfalls in Gegenwart eines Verdünnungsmittels, einer Base oder eines Reaktionsbeschleunigers umsetzt, wobei $R^4$ die imAnspruch 1 genannten Bedeutungen ausser der Hydroxylgruppe hat, und die so erhaltene Verbindung gegebenenfalls schwefelt.

6. Herbizid gemäss Anspruch 1, enthaltend eine 2-Fluoralkancarbonsäure-Verbindung, nämlich 2-(2', 4'-Dichlorphenoxy)- 2-fluorpropionsäure, 2-(2', 4'-Dichlorphenoxy)- 2-fluorpropionsäuremethylester, 2-(2', 4'-Dichlorphenoxy)-2-fluorpropionsäureisooctylester, 2-(3'-Trifluormethylphenoxy)-2-fluorpropionsäuremethylester, 2-(4'-Fluorphenoxy)-2-fluorpropionsäuremethylester, 2-(2'-Methyl-4'-chlorphenoxy)-2-fluorpropionsäure oder 2-(2',4'-Dichlorphenoxy)-2-fluorpropionsäure-2''-(3''-chlorphenoxy)-ethylester.

**Revendications pour l'Etats contractants: BE, CH, LI, DE, FR, GB, IT, LU, NL, SE**

1. Dérivé d'acide 2-fluoroalcanecarboxylique de formule générale

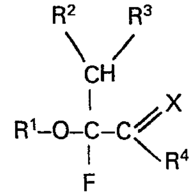

dans laquelle

$R^1$ représente un reste alkyle à chaîne droite de 1 à 14 atomes de carbone, éventuellement substitué par halogène ou nitro, un reste alkyle ramifié ou cyclique de 3 à14 atomes de carbone, un reste alcoxy ou alkylthio de 1 à 5 atomes de carbone, un reste halogénalkyle, halogénalcoxy, halogénalkyl thio ou halogénalkylsulfonyle ayant jusqu'à 4 atomes de carbone et jusqu'à 9 atomes d'halogène, un groupe formyl-, cyano- ou thiocyanate, un reste alcoxycarbonyle dialkylcéto, alkylcarbonyle, alkyl- et dialkylcarbamoyle, acyalmino-, ou alkylaminosulfonyle de 2 à 10 atomes de carbone, un reste alcoxyalkyle ou alkylthioalkyle de 2 à 6 atomes de carbone, un reste phényle ou phénylcarbonyle de 6 à 11 atomes de carbone, un reste aryloxy, arylalkyloxy ou arylazo, arylthio, arylsulfinyle ou arylsulfonyle, éventuellement substitué par chlore ou trifluorométhyle, un reste arylalkyle, un reste alcényle à chaîne droite de 2 à 6 atomes de carbone ou un reste alcényle à chaîne ramifiée de 3 à 7 atomes de carbone, un reste alcinyle à chaîne droite de 2 à 4, ou à chaîne ramifiée de 3 à 6 atomes de carbone, un reste aryle ou hétéroaryle condensé, substitué par un reste benzo, benzofurane ou pyridino

$R^2$ et $R^3$ représentent, indépendamment l'un de l'autre, hydrogène, un reste aryle ou hétéroaryle, éventuellement substitué comme pour $R^1$, ou un reste alkyle, éventuellement substitué par chlore, brome, fluor, nitro, cyano, méthoxy ou éthoxy ou un reste cycloalkyle éventuellement substitué par chlore ou trifluorométhyle, ou un reste aralkyle,

X représente oxygène ou soufre, et

$R^4$ représente un groupe hydroxyle ou les groupes $OR^5$, $SR^5$ ou

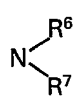 dans lesquels

$R^5$ représente un reste aryle ou hétéroaryle éventuellement substitué comme pour $R^1$ ou un reste alkyle éventuellement substitué par chlore, fluor, brome, méthoxy, éthoxy, butoxy, cyclohexoxy, méthylmercapto ou éthylmercapto, un reste alcényle éventuellement substitué par chlore, un reste cycloalkyle, un reste cycloalcényle, un reste alcanone ou un reste hétéro-arylalkyle,

$R^6$ représente hydrogène ou les restes indiqués pour $R^5$, outre un reste m-anilino éventuellement substitué,

$R^7$ représente, indépendamment de $R^6$, les res-

tes indiqués pour $R^5$ ou un groupe hydroxyle ou un reste alcoxy ou alkylthio, un groupe cyano ou cyanoalkyle ou le reste

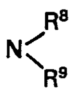 dans laquelle

$R^8$ et $R^9$ représentent, indépendamment l'un de l'autre, hydrogène ou un reste aryle éventuellement substitué comme pour $R^1$ou un reste alkyle et, en outre, $R^6$ et $R^7$ représentent, ensemble avec l'atome d'azote voisin, un reste hétérocyclique éventuellement substitué par méthyle, et qui contient éventuellement une ou plusieurs doubles liaisons ou contient, en plus de l'atome d'azote, un ou plusieurs hétéroatomes – ou les sels de ce composé.

2. Herbicide contenant un dérivé d'acide 2-fluoroalcanecarboxylique selon la revendication 1.

3. Herbicide contenant un véhicule solide ou liquide et un dérivé d'acide 2-fluoroalcanecarboxylique selon la revendication 1.

4. Procédé de préparation d'un herbicide caractérisé par le fait qu'on mélange un véhicule solide ou liquide avec un dérivé d'acide 2-fluoroalcanecarboxylique selon la revendication 1.

5. Procédé de lutte contre les plantes indésirables caractérisé par le fait qu'on traite les plantes ou le sol avec un dérivé d'acide 2-fluoroalcanecarboxylique selon la revendication 1.

6. Procédé de préparation d'un dérivé d'acide 2-fluoroalcanecarboxylique selon la revendication 1, caractérisé par le fait qu'on fait réagir

a) un dérivé d'acide acrylique de formule générale

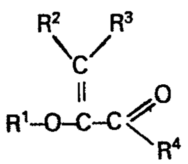 II

dans laquelle $R^1$, $R^2$, $R^3$ et $R^4$ ont les significations indiquées dans la revendication 1 avec de l'acide fluorhydrique, avec ou sans diluant ou un catalyseur, et l'on sulfure éventuellement le composé obtenu, ou

b) un acide 2-fluoroalcanecarboxylique de formule

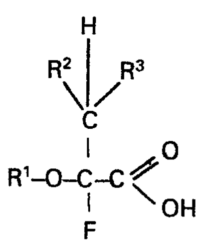

dans laquelle $R^1$, $R^2$ et $R^3$ ont les significations indiquées dans la revendication 1 ou leurs sels, avec du chlorure de thionyle, phosgène, pentachlorure de phosphore, N,N'-carbonyldiimidazole, N,N'-carbonylditriazole, N,N'-thionyldiimidazole ou N,N'-thionyltriazole, ou leur mélange, éventuellement en présence d'un diluant, puis on fait réagir avec un composé de formule $HR^4$, éventuellement en présence d'un diluant, d'une base ou d'un accélérateur de réaction, $R^4$ ayant la signification indiquée dans la revendication1, en plus du groupe hydroxyle et l'on sulfure éventuellement le composé obtenu.

7. Dérivé d'acide 2-fluoroalcanecarboxylique selon la revendication 1, notamment cide 2-(2',4'-dichlorophénoxy)-2-fluoropropionique, ester méthylique d'acide 2-(2',4'-dichlorophénoxy)-2-fluoropropionique, ester issoctylique d'acide 2-(2',4'-dichlorophénoxy)-2-fluoropropionique, ester méthylique d'acide 2-(3'-trifluorométhylphénoxy)-2-fluoropropionique, ester méthylique d'acide 2-(4'-fluorophénoxy)-2-fluoropropionique, acide 2-(2'-méthyl-4-chlorophénoxy)-2-fluoropropionique, ou ester 2''-(3''-chlorophénoxy)éthylique d'acide 2-(2',4'-doichlorophénoxy)-2-fluoropropionique.

8. Herbicide selon la revendication 2, contenant un dérivé d'acide 2-fluoroalcanecarboxylique, notamment acide 2-(2',4'-dichlorophénoxy)-2-fluoropropionique, ester méthylique d'acide 2-(2',4'-dichlorophénoxy)-2-fluoropropionique, ester isooctylique d'acide 2-(2',4'-dichlorophénoxy)-2-fluoropropionique, ester méthylique d'acide 2-(3'-trifluorométhylphénoxy)- 2-fluoropropionique, ester méthylique d'acide 2-(4'-fluorophénoxy)-2-fluoropropionique, acide 2-(2'-méthyl)-4'-chlorophénoxy)-2-fluoropropionique, ou ester 2''-(3''-chlorophénoxy)éthylique d'acide 2-(2', 4'-dichlorophénoxy)-2-fluoropropionique.

**Revendicatons pour l'Etat contractant: AT**

1. Herbicide contenent un dérivé d'acide 2-fluoroalcanecarboxylique de formule générale dans laquelle

$R^1$ représente un reste alkyle à chaîne droite de 1 à 14 atomes de carbone, éventuellement substitué par halogène ou nitro, un reste alkyle ramifié ou cyclique de 3 à 14 atomes de carbone, un reste alcoxy ou alkylthio de 1 à 5 atomes de carbone, un reste halogénalkyle, halogénalcoxy, halogénalkylthio ou halogénalkylsulfonyle ayant jusqu'à 4 atomes de carbone et jusqu'à 9 atomes d'halogène, un groupe formyl-, cyano- ou thiocyanate, un reste alcoxycarbonyle dialkylcéto, alkylcarbonyle, alkyl- et dialkylcarbamoyle, acylamino-, ou alkylaminosulfonyle de 2 à 10 atomes de carbone, un reste alcoxyalkyle ou alkylthioalkyle de 2 à 6 atomes de carbone, un reste phényle ou phénylcarbonyle de 6 á 11 atomes de carbone, un reste aryloxy, arylalkyloxy ou arylazo, arylthio, arylsulfinyle ou arylsulfonyle, éventuellement substitué par chlore ou trifluorométhyle, un reste arylalkyle, un reste arylalkyle, un reste alcényle à chaîne droite de 2 à 6 atomes de carbone ou un reste alcényle à chaîne ramifiée de 3 à 7 atomes de carbone, un reste alcinyle à chaîne droite de 2 à 4, ou à chaîne ramifiée de 3 à 6 atomes de carbone, un reste aryle ou hétéroaryle condensé, substitué par un reste benzo, benzofurane ou pyridino

$R^2$ et $R^3$ représentent, indépendamment l'un de l'autre, hydrogène, un reste aryle ou hétéroaryle,

éventuellement substitué comme pour $R^1$, ou un reste alkyle, éventuellement substitué par chlore, brome, fluor, nitro, cyano, méthoxy ou éthoxy ou un reste cycloalkyle éventuellement substitué par chlore ou trifluorométhyle, ou un reste aralkyle,

X représente oxygène ou soufre, et

$R^4$ représente un groupe hydroxyle ou les groupes $OR^5$, $SR^5$ ou

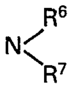 dans lesquels

$R^5$ représente un reste aryle ou hétéroaryle éventuellement substitué comme pour $R^1$ ou un reste alkyle éventuellement substitué par chlore, fluor, brome, méthoxy, éthoxy, butoxy, cyclohexoxy, méthylmercapto ou éthylmercapto, un reste alcényle, éventuellement substitué par chlore, un reste alcinyle éventuellement substitué par chlore, un reste cycloalkyle, un reste cycloalcényle, un reste alcanone ou un reste hétéro-arylalkyle,

$R^6$ représente hydrogène ou les restes indiqués pour $R^5$, outre un reste m-anilino éventuellement substitué,

$R^7$ représente, indépendamment de $R^6$, les restes indiqués pour $R^5$ ou un groupe hydroxyle ou un reste alcoxy ou alkylthio, un groupe cyano ou cyanoalkyle ou le reste

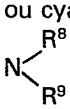 dans laquelle

$R^8$ et $R^9$ représentent, indépendamment l'un de l'autre, hydrogène ou un reste aryle éventuellement substitué comme pour $R^1$ ou un reste alkyle et, en outre, $R^6$ et $R^7$ représentent ensemble avec l'atome d'azote voisin un reste hétérocyclique éventuellement substitué par méthyle, et qui contient éventuellement une ou plusieurs doubles liaisons au contient, en plus de l'atome d'azote, un ou plusieurs hétéroatomes ou les sels de ce composé.

2. Herbicide contenant un véhicule solide ou liquide et un dérivé d'acide 2-fluoroalcanecarboxylique selon la revendication 1.

3. Procédé de préparation d'un herbicide caractérisé par le fait qu'on mélange un véhicule solide ou liquide avec un dérivé d'acide 2-fluoroalcanecarboxylique selon la revendication 1.

4. Procédé de préparation d'un herbicide caractérisé par le fait qu'on mélange un véhicule solide ou liquide avec un dérivé d'acide 2-fluoroalcanecarboxylique selon la revendication 1.

5. Procédé de préparation d'un dérivé d'acide 2-fluoroalcanecarboxylique selon la revendication 1, caractérisé par le fait qu'on fait réagir

a) un dérivé d'acide acrylique de formule générale

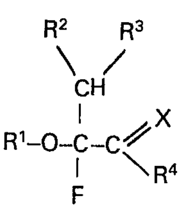

II

dans laquelle $R^1$, $R^2$, $R^3$ et $R^4$ ont les significations indiquées dans la revendication 1 avec de l'acide fluorhydrique, avec ou sans diluant ou un catalyseur, et l'on sulfure éventuellement le composé obtenu, ou

b) un acide 2-fluoroalcanecarboxylique de formule

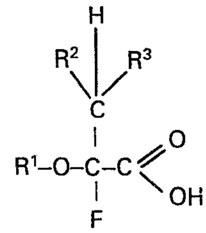

dans laquelle $R^1$, $R^2$ et $R^3$ ont les significations indiquées dans la revendication 1 ou leur sels, avec du chlorure de thionyle, phosgène, pentachlorure de phosphore, N,N'-carbonyldiimidazole, N,N'-carboylditriazole, N,N'-thionyldiimidazole ou N,N'-thionyltriazole, ou leur mélange, éventuellement en présence d'un diluant, puis on fait réagir avec le compé de formuel $HR^4$, éventuellement en présence d'un diluant, d'une base ou d'un accélérateur de réaction, $R^4$ ayant la signification indiquée dans la revendication 1, en plus du groupe hydroxyle et l'on sulfure éventuellement le composé obtenu.

6. Herbicide selon la revendication 1, contenant un dérivé d'acide 2-fluoroalcanecarboxylique, notamment acide 2-(2',4'-dichlorophénoxy)-2-fluoropropionique, ester méthylique d'acide 2-(2',4'-dichlorophénoxy)-2-fluoropropionique, ester isooctilique d'acide 2-(2',4'-dichlorophénoxy)-2-fluoropropionique, ester méthylique d'acide 2-(3'-trifluorométhylphénoxy)-2-fluoropropionique, ester méthylique d'acide 2-(4'-fluorophénoxy)-2-fluoropropionique, acide 2-(2'-méthyl-4'-chlorophénoxy)-2-fluoropropionique, ou ester 2''-(3''-chlorophénoxy)éthylique d'acide 2-(2',4'-dichlorophénoxy)-2-fluoropropionique.

**Claims for the Contracting states: BE, CH, LI, DE, FR, GB, IT, LU, NL, SE**

1. A 2-fluoroalkanecarboxylic acid compound of the general formula

where

$R^1$ is aryl or hetaryl optionally substituted by halogen, nitro, straight-chain alkyl of 1 to 14 carbon atoms, branched alkyl of 3 to 14 carbon atoms, cycloalkyl of 3 to 14 carbon atoms, alkoxy or alkylthio of 1 to 5 carbon atoms, haloalkyl, haloalkoxy, haloalkylthio or haloalkylsulfonyl of up to 4 carbon atoms and up to 9 halogen atoms, formyl, cyano or thiocyanato, alkoxycarbonyl, dialylketo, alkylcarbonyl, alkylcarbamyl, dialkylcarbamyl, acylamino or alkylaminosulfonyl of 2 to 10 carbon atoms, alkoxyalkyl or alkylthioalkyl of 2 to 6 carbon atoms, phenyl or phenylcarbonyl of 6 to 11 carbon atoms, aryloy, arylalkoxy or arylazo optionally substituted by chlorine or trifluoromethyl, arylthio, arylsulfinyl or arylsulfonyl, arylalkyl,

straight-chain alkenyl of 2 to 6 carbon atoms or branched alkenyl of 3 to 7 carbon atoms, straight-chain alkynyl of 2 to 4 carbon atoms or branched alkynyl of 3 to 6 carbon atoms, or a fused-on benzo, benzofuran or pyridino, $R^2$ and $R^3$, independently of one another, are hydrogen, aryl or hetaryl optionally bearing the same substituents as in the case of $R^1$; alkyl optionally substituted by chlorine, bromine, fluorine, nitro, cyano, methoxy or ethoxy; cycloalkyl optionally substitued by chlorine or trifluoromethyl; or aralkyl,

X is oxygen or sulfur,

$R^4$ is hydroxyl or is $OR^5$, $SR^5$ or 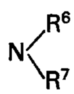 where

$R^5$ is aryl or hetaryl optionally bearing the same substituents as in the case of $R^1$; alkyl optionally substituted by chlorine, fluorine, bromine, methoxy, ethoxy, butoxy, cyclohexoxy, methylmercapto or ethylmercapto; alkenyl optionally substituted by chlorine; alkynyl optionally substituted by chlorine; cycloalkyl; cycloalkenyl; alkanonyl; or hetarylalkyl,

$R^6$ is hydrogen or has one of the meanings given for $R^5$, except optionally substituted n-anilino,

$R^7$, independently of $R^6$, has one of the meanings given for $R^5$ or is hydroxyl, alkoxy, alkylthio, cyano, cyanoalkyl or

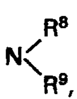 where

$R^8$ and $R^9$, independently of one another, are hydrogen; aryl optionally bearing the same substituents as in the case of $R^1$; alkyl, and

$R^6$ and $R^7$ together with the adjacent nitrogen can also be a heterocyclic radical which is optionally substituted by methyl and may or may not contain one or more double bonds and one or more hetero atoms in addition to the nitrogen, or a salt of this compound.

2. A herbicide containing a 2-fluoroalkanecarboxylic acid compound as claimed in claim 1.

3. A herbicide containing a solid or liquid carrier and a 2-fluoroalkanecarboxylic acid compound as claimed in claim 1.

4. A process for producing a herbicide, wherein a solid or liquid carrier is mixed with a 2-fluoroalkanecarboxylic acid compound as claimed in claim 1.

5. A process for combating unwanted plants, wherein the plants or the soil are treated with a 2-fluoroalkanecarboxylic acid compound as claimed in claim 1.

6. A process for preparing a 2-fluoroalkanecarboxylic acid compound as claimed in claim 1, wherein

a) an acrylic acid compound of the general formula

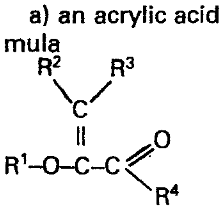

II,

where $R^1$, $R^2$, RU3 and $R^4$ have the meanings given in claim 1, is reacted with hydrofluoric acid, in the presence or absence of a diluent or a catalyst, with or without subsequent sulfurizing of the compound thus obtained, or

b) a 2-fluoroalkanecarboxylic acid of the formula

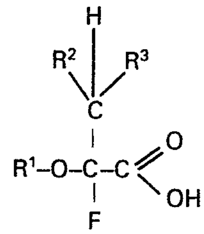

where $R^1$, $R^2$ and $R^3$ have the meanings given in claim 1, or a salt thereof, is reacted with thionyl chloride, phosgene, phosphorus pentachloride, N,N'-carbonyldiimidazole, N,N'-carbonylditriazole, N,N'-thionyldiimidazole or N,N'-thionylditriazole or a mixture of these, in the presence or absence of a diluent, and the product is then reacted with a compound of the formula

$HR^4$,

where $R^4$ has the meanings given in claim 1 other than hydroxyl, in the presence or absence of a diluent, a base or a reaction accelerator, with or without subsequent sulfurizing of the compound thus obtained.

7. A 2-fluoroalkanecarboxylic acid compound as claimed in claim 1, namely 2-(2',4'-dichlorophenoxy)-2-fluoropropionic acid, methyl 2-(2',4'-dichlorophenoxy)-2-fluoropropionate, isooctyl 2-(2',4'-dichlorophenoxy)-2-fluoropropionate, methyl 2-(3'-trifluoromethylphenoxy)-2-fluoropropionate, methyl 2-(4'-fluorophenoxy)-2-fluoropropionate, 2-(2'-methyl-4'- chlorophenoxy)-2-fluoropropionic acid or 2''-(3''-chlorophenoxy)-ethyl 2-(2',4'-dichlorophenoxy)-2-fluoropropionate.

8. A herbicide, as claimed in claim 2, containing a 2-fluoroalkanecarboxylic acid compound, namely 2-(2',4'-dichlorophenoxy)-2-fluoropropionic acid, methyl 2-(2',4'-dichlorophenoxy)-2-fluoropropionate, isooctyl 2-(2',4'-dichlorophenoxy)-2-fluoropropionate, methyl 2-(3'-trifluoromethylphenoxy)-2-fluoropropionate, methyl 2-(4'-fluorophenoxy)-2-fluoropropionate, 2-(2'-methyl-4'-chlorophenoxy)-2-fluoropropionic acid or 2''-(3''-chlorophenoxy)-ethyl 2-(2',4'-dichlorophenoxy)-2-fluoropropionate.

**Claims for the contracting state: AT**

1. A herbicide containing a 2-fluoroalkanecarboxylic acid compound of the general formula

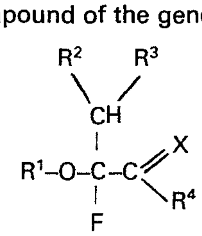

where

$R^1$ is aryl or hetaryl optionally substituted by halogen, nitro, straight-chain alkyl of 1 to 14 carbon atoms, branched alkyl of 3 to 14 carbon atoms, cycloalkyl of 3 to 14 carbon atoms, alkoxy of alkylthio of 1 to 5 carbon atoms, haloalkyl, haloalkoxy, haloalkylthio or haloalkylsulfonyl of up to 4 carbon atoms and up to 9 halogen atoms, formyl, cyano or thiocyanato, akkoxycarbonyl, dialkylketo, alkylcarbonyl, alkylcarbamyl, dialkylcarbamyl, acylamino or alkylaminosulfonyl of 2 to 10 carbon atoms, alkoxyalkyl or alkylthioalkyl of 2 to 6 carbon atoms, phenyl of phenylcarbonyl of 6 to 11 carbon atoms, aryloxy, arylalkoxy or arylazo optionally substituted by chlorine or trifluoromethyl, arylthio, arylsulfinyl or arylsulfonyl, arylalkyl, straightchain alkenyl of 2 to 6 carbon atoms or branched alkenyl of 3 to 7 carbon atoms, straightchain alkynyl of 2 to 4 carbon atoms or branched alkynyl of 3 to 6 carbon atoms, or a fused-on benzo, benzofuran or pyridino.

$R^2$ and $R^3$, independently of one another, are hydrogen; aryl or hetaryl optionally bearing same substituents as in the case of $R^1$; alkyl optionally substituted by chlorine, bromine, fluorine, nitro, cyano, methoxy or ethoxy; cycloalkyl optionally substituted by chlorine or trifluoromethyl; or aralkyl,

X is oxygen or sulfur,

$R^4$ is hydroxyl or is $OR^5$, $SR^5$ or 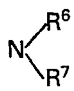 where

$R^5$ is aryl or hetaryl optionally bearing the same substituents as in the case of $R^1$; alkyl optionally substituted by chlorine, fluorine, bromine, methoxy, ethoxy, butoxy, cyclohexoxy, methylmercapto or ethylmercapto; alkenyl optionally substituted by chlorine; alkynyl optionally substituted by chlorine; cycloalkyl; cycloalkenyl; alkynonyl; or hetarylalkyl, $R^6$ is hydrogen or has one of the meanings given for $R^5$, except optionally substituted n-anilino,

$R^7$, independently of $R^6$, has one of the meanings given for $R^5$ or is hydroxyl, alkoxy, alkylthio, cyano, cyanoalkyl or

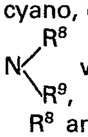 where

$R^8$ and $R^9$, independently of one another, are hydroger; aryl optionally bearing the same substituents as in the case of $R^1$; alkyl, and

$R^6$ and $R^7$ together with the adjacent nitrogen can also be a heterocyclic radical which is optionally substituted by methyl and may or may not contain one or more double bonds and one or more hetero atoms in addition to the nitrogen, or a salt of this compound.

2. A herbicide containing a solid or liquid carrier and a 2-fluoroalkanecarboxylic acid compound as defined in claim 1.

3. A process for producing a herbicide, wherein a solid or liquid carrier is mixed with a 2-fluoroalkanecarboxylic acid compound as defined in claim 1.

4. A process for combating unwanted plants, wherein the plants or the soil are treated wich a 2-fluoroalkanecarboxylic acid compound as defined in claim 1.

5. A process for preparing a 2-fluoroalkanecarboxylic acid compound as defined in claim 1, wherein

a) an acrylic acid compound of the general formula

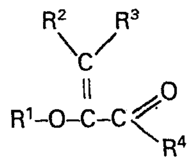 II

where $R^1$, $R^2$, $R^3$ and $R^4$ have the meanings given in claim 1, is reacted with hydrofluoric acid, in the presence or absence or a diluent or a catalyst, with or without subsequent sulfurizing of the compound thus obtained, or

b) a 2-fluoroalkanecarboxylic acid of the formula

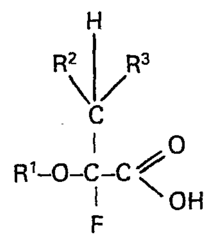

where $R^1$, $R^2$ and $R^3$ have the meanings given in claim 1, or a salt thereof, is reacted with thionyl chloride, phosgene, phosphorus pentachloride, N,N'-carbonyldiimidazole, N,N'-carbonylditriazole, N,N'-thionyldiimidazole or N,N'-thionyldiimidazole or N,N'-thionylditriazole or a mixture of these, in the presence or absence or a diluent, and the product is then reacted with a compound of the formula

$$HR^4,$$

Where $R^4$ has the meanings given in claim 1 other than hydroxyl, in the presence or absence of a diluent, a base or a reaction accelerator, with or without subsequent sulfurizing of the compound thus obtained.

6. A herbicide, as claimed in claim 1, containing a 2-fluoroalkanecarboxylic acid compound, namely 2-(2',4'-dichlorophenoxy)-2-fluoropropionic acid, methyl 2-(2',4'-dichlorophenoxy)-2-fluoropropionate, isooctyl 2-(2',4'-dichlorophenoxy)-2-fluoropropionate, methyl 2-(3'-trifluoromethylphenoxy)-2-fluoropropionate, methyl 2-(4'-fluorophenoxy)-2-fluoropropionate, 2-(2'-methyl-4-chlorophenoxy)-2-fluoropropionic acid or 2''-(3''-chlorophenoxy)-ethyl 2-(2',4'-dichlorophenoxy)-2-fluoropropionate.